# FASCICULE DE BREVET EUROPEEN

(11) **EP 2 185 531 B1**
(45) Date de publication et mention de la délivrance du brevet: **25.07.2012**
(21) Numéro de dépôt: 08836682.8
(22) Date de dépôt: 05.09.2008
(51) Int. Cl.: C07D 279/20, A01N 43/84

(54) **Composés diaminophénothiazine, leur procédé de préparation et leurs utilisations**
Diaminophenothiazinverbindungen, Verfahren zu ihrer Herstellung und Verwendungen davon
Diaminophenothiazine compounds, a method for preparing same and uses thereof

(30) Priorité: 07.09.2007 FR 0757406
(43) Date de publication de la demande: 19.05.2010
(73) Titulaire: Pharma Hydro Development - P.H.D., 51000 REIMS (FR)
(72) Inventeur: GALEY, Laurent, F-77184 EMERAINVILLE (FR); PETERS, Fabrice, F-75007 PARIS (FR); FERRY, Xavier, F-67000 STRASBOURG (FR)
(74) Mandataire: Catherine, Alain
(86) Numéro de dépôt international: PCT/FR2008/051588
(87) Numéro de publication internationale: WO 2009/044054

(56) Documents cités:
- WO-A-2005/054217
- WO-A-2007/110627
- US-A- 5 220 009
- J. G. MICHELS ET AL.: "Studies in the sulfone series." JOURNAL OF THE AMERICAN CHEMICAL SOCIETY., vol. 72, février 1950 (1950-02), pages 888-892, XP002478603 USAMERICAN CHEMICAL SOCIETY, WASHINGTON, DC.

## Description

### Domaine de l'invention

La présente invention se rapporte à de nouveaux composés diaminophénothiazine ainsi qu'à leur procédé de préparation.

L'invention concerne aussi les utilisations des nouveaux composés diaminophénothiazine dans le domaine du traitement des fluides, liquides ou gazeux, notamment des effluents industriels ou domestiques.

### Art antérieur

De manière générale, il existe un besoin constant dans l'état de la technique pour de nouveaux agents biocides utilisables dans des domaines industriels variés où l'utilisation de tels agents biocides et oxydants est requise, y compris dans les domaines agro-alimentaires et du traitement des effluents.

Le risque microbiologique est une réalité redoutable, parce que changeante. Les techniques et moyens mis en oeuvre sélectionnent inéluctablement des populations résistantes à ces techniques et moyens, ou font émerger des pathologies non documentées ou anciennes. Les systèmes, actuels en stérilisation et-ou désinfection de matières, se comportent comme des concentrateurs d'agents vivants et de molécules résistantes à ces mêmes traitements, mettant en risque la santé humaine, animale, et végétale (affluents), et comme des réservoirs de ces mêmes produits et risques (effluents).

Les produits et techniques biocides permettant de limiter ou empêcher le risque microbiologique sont anciens, que ce soit pour l'hygiène générale (désinfection, potabilisation, épuration), ou le traitement des pathologies et /ou désagréments liés à cette population.

Les industries fines ou lourdes, type pharmacie, agro-alimentaire, microélectronique, doivent produire dans des conditions de stérilité et/ou de pureté élevées.

Le traitement et l'épuration des eaux, sont des processus de plus en plus complexes est onéreux, vu la dégradation des niveaux de salubrité, chimique ou microbiologique, des ressources naturelles.

La sophistication croissante des gestes invasifs en santé humaine et animale impose une désinfection totale des affluents (eau, gaz, atmosphères). Le traitement des affluents et effluents est en 2007 mal maîtrisé.

Le nombre des infections nosocomiales reste et demeure élevé.

La forte sélection des désinfectants classiques (agents oxydants, biguanides, ammoniums quaternaires, etc), permet de limiter les microorganismes pathogènes humains classiques, mais sélectionne et donc introduit dans le circuit des affluents, des microorganismes de diverses origines, par exemple biotelluriques (*Pseudomonas* spp. *Clostridrium* spp., *Cryptosporidium* spp., etc). Ces nouveaux agents se révèlent très difficiles à traiter et à éradiquer.

Quant aux antibiotiques, leur utilisation permanente fait effondrer leurs spectres d'action sur les bactéries pathogènes classiques.

Comme déjà indiqué, on utilise fréquemment des agents désinfectants dans les procédés de traitement d'effluents industriels ou domestiques. Illustrativement, les procédés de traitement des effluents, qu'ils consistent en des effluents domestiques ou industriels, comprennent généralement les étapes successives suivantes :
- une étape de relevage permettant d'alimenter l'installation en effluents à traiter :
- une étape de dégrillage destinée à débarrasser les effluents à traiter des polluants solides les plus grossiers, par séparation physique,
- une étape dite de « traitement primaire » consistant en une étape de séparation physique permettant d'éliminer la majorité des matières en suspension, cette étape consistant en général en une étape de séparation physique par décantation. Cette étape de séparation par décantation est couramment réalisée avec la provocation d'une floculation préalable de polluants. La floculation des polluants est réalisée par ajout, dans le dispositif de décantation, d'agents floculants, par exemple des sels métalliques, induisant la précipitation (floculation) des MES (Matières en Suspension) ainsi que de certains polluants organiques et minéraux.
- une étape dite de « traitement secondaire » principalement destinée à l'élimination des polluants carbonés ou azotés, qui mettent généralement en oeuvre des procédés biologiques utilisant des bactéries éventuellement immobilisées sur des supports, y compris des membranes filtrantes ;
- une étape de désinfection destinée à détruire les organismes uni-cellulaires oumulti-cellulaires indésirables, y compris les micro-organismes indésirables, qui peut être réalisée par ajout d'un agent biocide aux effluents traités, avant leur rejet dans l'environnement naturel. L'étape de désinfection est couramment réalisée par ajout de chlore. Cette étape de désinfection par des agents oxydants, peut être aussi réalisée en ajoutant des agents biocides comme l'ozone ou le brome, et dans certains cas du dioxyde de chlore. Alternativement, l'étape de désinfection peut être réalisée par exposition des effluents au rayonnement solaire ou encore à un rayonnement ultraviolet artificiel, afin de détruire les organismes vivant indésirables ;
- une étape de clarification des effluents, qui consiste généralement en en une ultime étape de décantation permettant de séparer l'effluent épuré et les boues ou résidus secondaires issus de la dégradation des matières organiques, avant rejet de l'effluent épuré et désinfecté dans l'environnement naturel.

Les effluents d'origine humaine ou animale présentent une charge microbiologique moyenne de 10⁵ à 10⁷ bactéries/ml revivifiables à 22°C. En station d'épuration, les microorganismes issus des tractus digestifs humains et animaux, peuvent interférer avec la population bactérienne normale des eaux. Il en résulte un échange et transfert de matériel génétique, dont les gènes de résistance aux antibiotiques. Des bactéries, qui passent les barrières chlore-ozone, et normalement présentes dans l'eau potable des réseaux et des sources, deviennent donc résistantes aux antibiotiques. Le processus inverse est possible ; boire de l'eau potable (traitée ou de source) peut induire le même transfert vers les bactéries intestinales normales. Le cercle vicieux est complet

Une méthode permettant de générer in situ des produits hautement réactifs est connue en synthèse organique. Il s'agit de l'action photochimique, capable de générer des états réactifs de molécules organiques, et notamment de l'oxygène singulet à partir de l'oxygène triplet, sous l'influence d'un photon. Les molécules capables d'une telle action sont dites photosensibilisateurs ; l'énergie photonique excite le photosensibilisateur, qui transfère cette énergie sur une autre molécule. Dans le cas où cette molécule est de l'oxygène, la molécule passe de l'état triplet, peu réactive, à l'état singulet, très réactive (réaction de type II, C.S.Foote, Photochem.Photobiol.,1991 : 54,659). Cet oxygène singulet est une forme non-radicalaire, capable de former des radicaux anions superoxyde, eux-mêmes hautement réactifs.

La chimie organique utilise divers photosensibilisateurs pour générer des radicaux à partir de molécules neutres, des dérivés carbonylés (acétone, benzophénone, ménadione) et des colorants (rose bengale, bleu de méthylène)

La série du bleu de méthylène (diamino 3,7-phénothiazine, voir supra) est particulièrement intéressante. Cette molécule (et ses dérivés) est un colorant histologique classique, peu toxique, douée de diverses propriétés biologiques et pharmacologiques : antiseptique modéré (bactériostatique), antidote des intoxications aux nitrites et agents methémoglobinisants. Ses propriétés photoactivables (générateur d'oxygène singulet biocide) sont déjà utilisées comme agents stérilisants des produits dérivés du sang. À partir de la structure 3,7-diaminophénothiazine exclusivement, un grand nombre de dérivés ont été synthétisés. Divers auteurs ont démontré l'intérêt de ces produits lors de photoactivation, dans le traitement de pathologies cutanées, biocide général sur procaryotes et eucaryotes, etc...(Voir par exemple l'état de l'art dans WO 2005/054217 A1, US-A-5220009 2005/034855 A2).

Toutefois, les dérivés de la famille des 3,7-diaminophénothiazine consistent en des structures ioniques, sous la forme de composés ammoniums instables. Ces composés n'apportent que peu d'améliorations spécifiques au bleu de méthylène. De plus ces composés ne sont photoactivables qu'en rayonnement ultra violet

La Demanderesse a cherché à synthétiser de nouveaux agents biocides utilisables de manière générale comme agents désinfectants, y compris comme agents désinfectants dans des procédés de traitement des effluents pollués.

### Résumé de l'invention

La présente invention a pour objet de nouveaux composés biocides 2,8-diaminophénothiazine de formule (I) définis plus loin dans la présente description.

Cette invention a également pour un objet un procédé pour la préparation des composés 2,8-diaminophénothiazine de formule (I).

L'invention concerne aussi l'utilisation des nouveaux composés 2,8-diaminophénothiazine de formule (I) comme agents biocides.

### Description détaillée de l'invention

De nouveaux composés biocides de la famille des 2,8-diaminophénothiazine sont fournis selon l'invention.

A la connaissance du demandeur les seuls composés de la famille des 2,8-diaminophénothiazine connus sont respectivement le composé diaminé non substitué et son précurseur acylé. Ces produits ont été préparés par une première étape de diacétylation de la phénothiazine, suivie d'une seconde étape de formation de l'oxime et son réarrangement selon la réaction de Beckman, comme décrit par Chien et al. (J. Med. Chem., 1966, Vol. 9 : 960-962). On a aussi décrit un procédé comprenant une première étape de transformation de la dicétone en diacide, suivie d'une seconde étape d'activation de ces fonctions sous forme de chlorure d'acide, puis d'une étape de transformation du produit résultant en acylazide avant de réaliser une étape de transposition pour obtenir la diamine (Michels et al., 1950, J. Am. Chem. Soc., Vol. 72 : 888-892). Michels et al. (1950) décrivent aussi un procédé comprenant une étape de transformation du bromure de 2-bromomo-4-nitroaniline en le composé thiophénol correspondant. Puis le composé thiophénol est condensé sur le 3,4-diiodonitrobenzène, préalablement à une réaction de couplage d'Ullmann intramoléculaire permettant d'obtenir la 2,8-dinitrophénothiazine qui est ensuite réduite en 2,8-diaminophénothiazine. Chien et al. (1966, Supra) ne suggèrent pas de tester une quelconque activité biologique du composé 2,8-diaminophénothiazine, qui n'est qu'un composé intermédiaire pour l'obtention du produit final 2,8-diaminophénothiazine -5,5-dioxyde.

De manière surprenante, on a montré selon l'invention que ces nouveaux composés 2,8-diaminophénothiazine possèdent, après photoactivation, une activité biocide à l'encontre d'une grande variété de micro-organismes et qu'en conséquence ces nouveaux composés sont utiles en tant que biocides, et en particulier en tant qu'agents désinfectants. La (les) DAP-2,8 tétraméthyl (ou autres) présente(nt) d'une façon surprenante une activité floculante des protéines et/ou des composants de milieux de cultures bactériens, type extrait de levure ou albumine animale, jamais décrites sur les DAP 3-7 ni les 2-8 dioxamines antérieures

En milieu enrichi en protéines (Albumine bovine (BSA) ou extrait de levure) la DAP-2,8 tétraméthyl flocule spontanément les milieux, et se concentre dans le culot. La solution passe du jaune initial à un bleu-vert intense. Il existe un rapport dose /effet entre 0,3 et 10 g /I de protéine, avec précipitation du mélange proteines-bactéries-DAP. Cette propriété surprenante permet une solution globale du traitement des eaux usées, permettant une seule étape de floculation/décantation /désinfection. Ces produits, notamment sous forme de dichlorhydrates hydrosolubles, présentent un pouvoir bactéricide sur différentes souches de bactéries gram + (*Staphylococcus aureus*), et gram - (*E.coli, Pseudomonas aeruginosa Legionella pneumophila*) -. Le produit est également actif sur des eucaryotes filamenteux, par exemple *Candida albicans.* Ce spectre des DAP 2-8 (bactéricide sur gram - ) est totalement différent de celui des 3-7 diaminophénothiazines (bactériostatique strict sur gram +)

Selon des méthodes normées, la réduction du titre infectieux peut atteindre 8 log en 30 mm sous lumière blanche, soit 10000 fois plus que le bleu de méthylène témoin dans les mêmes conditions. Cette activité s'exerce dès la concentration de 0,05 g/l, soit 0,05 pour mille (poids/poids). Il existe un effet à l'obscurité, mais celui ci est plus tardif, au moins d'un facteur 2.

De plus, le demandeur a mis au point un procédé original pour la préparation de ces nouveaux composés 2,8-diaminophénothiazine biocides.

L'invention a pour objet un composé 2,8-diaminophénothiazine (DAP-2,8) de formule (I) suivante : 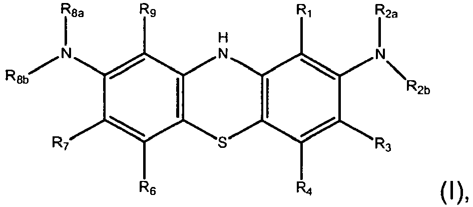 dans laquelle :
(i) les groupes R₁, R₃, R₄, R₆, R₇ et R₉ représentent chacun, indépendamment l'un de l'autre un groupe choisi parmi :
   - un atome d'hydrogène,
   - un halogène,
   - un groupe alkyle ayant de 1 à 12 atomes de carbone non substitué,
   - un groupe alkyle ayant de 1 à 12 atomes carbone substitué par un groupe phényle, ledit groupe phényle étant :
   - soit non substitué,
   - soit substitué par un ou plusieurs groupes choisis parmi un halogène, un hydroxy, un alkyle ayant de 1 à 12 atomes de carbone, et un alcoxy ayant de 1 à 12 atomes de carbone,
(ii) les groupes R₂ₐ, R_{2b}, R₈ₐ et R_{8b} représentent chacun, indépendamment l'un de l'autre un groupe choisi parmi :
   - - un atome d'hydrogène,
   - - un groupe alkyle, un groupe hydroxyalkyle ou un groupe alcoxyalkyle ayant de 1 à 12 atomes de carbone non substitué,
   - - un groupe alkyle ou un groupe alcoxyalkyle ayant de 1 à 12 atomes de carbone substitué par un groupe phényle, ledit groupe phényle étant :
   - - soit non substitué,
   - - soit substitué par un groupe choisi parmi
      - un halogène,
      - un hydroxy,
      - un alkyle ayant de 1 à 12 atomes de carbone,
      - un alcoxyalkyle ayant de 1 à 12 atomes de carbone,
      - un alcényle ayant de 2 à 12 atomes de carbone, non substitué ou substitué par un groupe choisi parmi un halogène, un hydroxy, un alkyle ayant de 1 à 12 atomes de carbone, un alcoxy ayant de 1 à 12 atomes de carbone,
      - un alcynyle ayant de 2 à 12 atomes de carbone, non substitué ou substitué par un groupe choisi parmi un halogène, un hydroxy, un alkyle ayant de 1 à 12 atomes de carbone, un alcoxy ayant de 1 à 12 atomes de carbone,
      - un cycloalkyle ayant de 4 à 12 atomes de carbone non substitué ou substitué par un groupe choisi parmi un halogène, un hydroxy, un alkyle ayant de 1 à 12 atomes de carbone, un alcoxy ayant de 1 à 12 atomes de carbone,
      - un cycloalcényle ayant de 4 à 12 atomes de carbone non substitué ou substitué par un groupe choisi parmi un halogène, un hydroxy, un alkyle ayant de 1 à 12 atomes de carbone, un alcoxy ayant de 1 à 12 atomes de carbone,
(iii) ou bien les groupes -NR₂ₐR_{2b} ou -NR₈ₐR_{8b} représentent, indépendamment l'un de l'autre, un hétérocycle ayant de 4 à 12 atomes de carbone, saturé ou insaturé, ledit hétérocycle pouvant comprendre un ou plusieurs hétéroatomes additionnels choisis parmi l'azote, l'oxygène et le soufre, ledit hétérocycle étant non substitué ou étant substitué par un groupe choisi parmi un halogène, un hydroxy et un alkyle ayant de 1 à 12 atomes de carbone,
ainsi que tout isomère optique, stéréoisomère, diastéréoisomère, énantiomère ou encore mélange racémique d'un composé de formule (I),
ainsi que les sels , les hydrates, les solvates et les formes polymorphes d'un composé de formule (I),
à l'exception des composés de formule (I) pour lesquels chacun des groupes choisis parmi R₁, R₂ₐ, R_{2b}, R₃, R₄, R₅, R₇, R₈ₐ, R_{8b} et R₉ représente un atome d'hydrogène.

Selon l'invention, l'atome d'halogène est choisi parmi les atomes de chlore, fluor, brome et iode.

Par « alkyle », on entend selon l'invention une chaîne linéaire ou ramifiée d'un radical hydrocarboné saturé monovalent et possédant le nombre d'atomes de carbone spécifié. Dans un groupe alkyle ramifié, la chaîne hydrocarbonée linéaire est substituée par un ou plusieurs groupes alkyle. Les groupes alkyles englobent les groupes méthyle, éthyle, propryle, butyle, pentyle et hexyle.

Par « alcoxy », on entend une chaîne linéaire ou ramifiée d'un radical alcool hydrocarboné saturé monovalent dérivant de l'élimination d'un atome d'hydrogène d'un groupe hydroxy. Typiquement, un groupe alcoxy a la formule générale « R-O- », avec R signifiant un groupe alkyle.

Par « alcoxyalkyle », on entend une chaîne alkyle interrompue par un atome d'oxygène et qui possède le nombre d'atomes de carbone spécifié. Typiquement, un groupe alcoxyalkyle a la formule générale « R-O-R'- », dans laquelle R et R' signifient chacun un groupe alkyle.

Par « alcényle », on entend une chaîne linéaire ou ramifiée d'un radical hydrocarboné monovalent, partiellement insaturé, possédant au moins une double liaison carbone-carbone et qui possède le nombre d'atomes de carbone spécifié. Les atomes liés aux atomes de carbones de la double liaison peuvent être orientés indifféremment dans une conformation cis (Z) ou en trans. Les groupes alcényle englobent les groupes éthènyle, propènyle et butènyle. Dans un groupe alcényle ramifié, la chaîne linéaire hydrocarbonée insaturée est substituée par un ou plusieurs groupes alkyle ou alcényle. Les groupes substituants alkyle englobent les groupes méthyle, éthyle ou propyle. Les groupes substituants alcényle englobent les groupes vinyle, allyle, et prop-1-ényle.

Par « alcynyle », on entend une chaîne linéaire ou ramifiée d'un radical monovalent, possédant au moins une triple liaison carbone-carbone et qui possède le nombre d'atomes de carbone spécifié. Les groupes alcynyle englobent les groupes éthynyle, propynyle et butynyle. Dans un groupe alcynyle ramifié, la chaîne linéaire insaturée est substituée par un ou plusieurs groupes alkyle, alcényle ou alcynyle. Les groupes substituants alkyle englobent les groupes méthyle, éthyle ou propyle. Les groupes substituants alcényle englobent les groupes vinyle, allyle, et prop-1-ényle. Les groupes substituants alcynyle englobent les groupes éthynyle, propargyle et prop-1-yn-1-yle.

Par « cycloalkyle », on entend un radical cyclique hydrocarboné monocyclique ou polycyclique saturé. Les groupes cycloalkyle englobent les groupes monocycliques cyclobutyle, cyclopentyle, cyclohexyle,cycloheptyle et cyclooctyle. Les groupes cycloalkyle englobent aussi les groupes polycycliques norbornane et decaline.

Par « cycloalcényle », on entend un radical cyclique hydrocarboné monocyclique ou polycyclique, partiellement insaturé, possédant au moins une double liaison carbone-carbone. Les groupes cycloalcényle englobent les groupes monocycliques cyclobutenyle, cyclopentenyle, cyclopentadiényle, cyclohexenyle, cycloheptenyle et cyclooctenyle. Les groupes cycloalcényle englobent aussi les groupes polycycliques adamantane et norbornène.

Par « hétérocycle », on entend un système monocyclique hydrocarboné saturé ou partiellement insaturé dans lequel au moins un atome de carbone est remplacé par un hétéroatome choisi parmi l'azote, l'oxygène et le soufre. Les groupes hétérocycliques englobent les groupes pyrrolidine, pipéridine, morpholine, thiomorpholine, 1,4-dioxa-8-azaspiro[4,5]décane et 1,2,3,6-tétrahydropyridine. Les groupes hétérocycliques englobent aussi les groupes de ce type décrits dans l'ouvrage « Handbook of Chemistry and Physics, 76th edition, CRC Press, Inc., 1995-1996 », pages 2-25à 2-26, cet extrait d'ouvrage étant incorporé par référence dans la présente description.

Le terme « substitué » se rapporte à un groupe de référence dans lequel au moins un des atomes d'hydrogène est remplacé par un groupe chimique distinct.

De manière générale, quel que soit le substituant concerné, un groupe alkyle ayant de 1 à 12 atomes de carbone consiste préférentiellement en un groupe alkyle ayant de 1 à 6 atomes de carbone.

Pour les groupes R₁, R₃, R₄, R₆, R₇ et R₉, le groupe alkyle, substitué ou non substitué, consiste préférentiellement en un groupe alkyle ayant de 1 à 6 atomes de carbone.

Pour les groupes R₁, R₃, R₄, R₆, R₇ et R₉ substitués par un groupe phényle, avec le groupe phényle substitué par un ou plusieurs groupes alkyle ou alcoxy, le groupe alkyle ou le groupe alcoxy de 1 à 12 atomes de carbone consiste préférentiellement en un groupe alkyle ou alcoxy ayant de 1 à 6 atomes de carbone.

Pour les groupes R₂ₐ, R_{2b}, R₈ₐ et R_{8b}, le groupe alkyle, hydroxyalkyle ou alcoxyalkyle, non substitué ou substitué, consiste préférentiellement en un groupe alkyle, hydroxyalkyle ou alcoxyalkyle ayant de 1 à 6 atomes de carbone.

Pour les groupes R₂ₐ, R_{2b}, R₈ₐ et R_{8b} substitués par un groupe phényle, le groupe phényle étant lui-même substitué par un groupe alkyle, ledit groupe alkyle a préférentiellement de 1 à 6 atomes de carbone.

Pour les groupes R₂ₐ, R_{2b}, R₈ₐ et R_{8b} substitués par un groupe phényle, le groupe phényle étant lui-même substitué par un groupe alcoxyalkyle, ledit groupe alcoxyalkyle a préférentiellement de 1 à 6 atomes de carbone.

Pour les groupes R₂ₐ, R_{2b}, R₈ₐ et R_{8b} substitués par un groupe phényle, le groupe phényle étant lui-même substitué par un groupe alcényle, ledit groupe alcényle a préférentiellement de 2 à 6 atomes de carbone.

Pour les groupes R₂ₐ, R_{2b}, R₈ₐ et R_{8b} substitués par un groupe phényle, le groupe phényle étant lui-même substitué par un groupe alcynyle, ledit groupe alcynyle a préférentiellement de 2 à 6 atomes de carbone.

Pour les groupes R₂ₐ, R_{2b}, R₈ₐ et R_{8b} substitués par un groupe phényle, le groupe phényle étant lui-même substitué par un groupe cycloalkyle, ledit groupe cycloalkyle a préférentiellement de 4 à 6 atomes de carbone.

Pour les groupes R₂ₐ, R_{2b}, R₈ₐ et R_{8b} substitués par un groupe phényle, le groupe phényle étant lui-même substitué par un groupe cycloalcényle, ledit groupe cycloalcényle a préférentiellement de 4 à 6 atomes de carbone.

Les divers isomères des composés de formule (I), y compris les isomères optiques, les énantiomères, les stéréoisomères et les diastéréoisomères sont définis du fait de la présence d'un ou plusieurs centres asymétriques dans la structure de ces composés, à partir des termes de la stéréochimie absolue, comme les diverses formes (R-) ou (S-) desdits centres asymétriques. Les divers isomères des composés de formule (I), y compris les isomères optiques, les énantiomères, les stéréoisomères et les diastéréoisomères sont préparés selon des méthodes bien connues de l'homme du métier (voir l'article "Molécules chirales Stéréochimie et propriétés" André COLLET, Jean CRASSOUS, Jean-Pierre DUTASTOU, Laure GUY - 01/01/2006 - EDP SCIENCES Isbn : 2-86883-849-9)

L'invention englobe les sels des composés de formule (I), y compris les sels d'addition avec des acides inorganiques tels que les chlorhydrates, les bromhydrates, les sulfates, les nitrates et les phosphates. L'invention englobe aussi les sels d'addition avec les acides organiques tels que les acétates, les propionates, les succinates, les maléates, les fumarates, les méthanesulfonates, les p-toluènesulfonates, et les iséthionates. Les sels de composés de formule (I) préférés consistent en les sels décrits par P.H. Stahl et C.G. Wermuth dans l'ouvrage « Handbook of Pharmaceutical salts, Properties, Selection and Use, Wiley-VCH, 2002 » et dans l'ouvrage « Remington's Pharmaceutical Sciences, 17th edition, Mack Publishing Company, Easton, PA, 1985 », page 1418.

L'invention englobe aussi les hydrates des composés de formule (I) qui peuvent par exemple être obtenus par :
(a) séchage des cristaux humides des composés de formule (I) à basse température, par exemple à une température allant de 20°C à 50°C, de préférence de 20°C à 40°C, et pendant une durée allant de 6 heures à 72 heures, de préférence de 12 heures à 24 heures, ou
(b) séchage des cristaux des composés de formule (I) à haute température, par exemple à une température allant de 80°C à 130°C, de préférence de 90°C à 120°C, puis exposer les cristaux secs à une atmosphère humide, par exemple une atmosphère ayant un degré d'humidité allant de 40% à 100% de saturation, et pendant une durée allant de 6 heures à 72 heures, de préférence de 12 heures à 24 heures.

La formation d'un hydrate d'un composé de formule (I) peut être confirmée en mesurant la teneur en eau de l'hydrate résultant, qui doit correspondre à la teneur en eau attendue dudit hydrate, par exemple en utilisant la méthode de Karl Fischer et en soumettant ledit hydrate à une mesure par Calorimétrie différentielle à balayage (ou DSC pour « Differential Scanning Calorimetry »), comme cela est bien connu de l'homme du métier.

De manière surprenante, les composés de formule (I) se présentent sous deux formes, respectivement sous forme de bases et sous forme de solvates, y compris lorsqu'ils sont sous la forme de sels, par exemple de sels avec des halogènes, ou avec des acides saturés ou non saturés, y compris des acides alkylcarboxyliques.

Les solvates des composés de formule (I) peuvent être préparés selon des méthodes conventionnelles bien connues de l'homme du métier, par exemple par dissolution d'un composé de formule (I) dans des solvants tels que l'eau, le méthanol ou l'éthanol, puis en recristallisant ledit composé en utilisant une technique de cristallisation également conventionnelle.

De manière générale, les composés de formule (I) peuvent être solvatés dans un milieu hydrophobe. Ces composés peuvent par exemple être immobilisés sur des supports, inertes ou non inertes, par exemple à la surface de membranes filtrantes, y compris les membranes filtrantes couramment utilisées pour la mise en oeuvre des procédé de traitement d'effluents industriels ou domestiques.

Les composés de formule (I) peuvent aussi être solvatés dans un milieu hydrophile, par exemple par ajout direct dans un fluide à traiter, tel que des eaux ou des fluides usés, ou encore des affluents ou des effluents d'origines variées. Les composés de formule (I) peuvent notamment être ajoutés dans des dispositifs de traitement en système ouvert ou fermé, par exemple dans des circuits de climatisation, de dialyse, de gaz, de désodorisation, d'élimination des COV ou micropolluants et tous autres produits sensibles à l'oxydation et susceptibles de l'être par ce procédé.

Les polymorphes d'un composé de formule (I) englobent les diverses formes cristallines de ces composés.

Dans certains modes de réalisation des composés de formule (I), les groupes R₂ₐ et R_{2b} représentent chacun un atome d'hydrogène.

Dans certains modes de réalisation des composés de formule (I), les groupes R₈ₐ et R_{8b} représentent chacun un atome d'hydrogène.

Dans certains modes de réalisation des composés de formule (I) pour lesquels les groupes R₂ₐ, R_{2b}, _{R8a} et R_{8b} représentent chacun, indépendamment l'un de l'autre un groupe alkyle, un groupe hydroxalkyle ou un groupe alcoxyalkyle ayant de 1 à 12 atomes de carbone substitué par un groupe phényle, ledit groupe phényle étant substitué par un groupe choisi parmi
- un halogène,
- un groupe alcényle, alcynyle, cycloalkyle ou cycloalcényle, ledit groupe étant substitué par un halogène,
ledit halogène étant préférentiellement un fluor.

Dans certains modes de réalisation des composés de formule (I) pour lesquels les groupes -NR₂ₐR_{2b} ou -NR₈ₐR_{8b} représentent, indépendamment l'un de l'autre, un hétérocycle ayant de 4 à 12 atomes de carbone, saturé ou insaturé, ledit hétérocycle pouvant comprendre un ou plusieurs hétéroatomes additionnels choisis parmi l'azote, l'oxygène et le soufre, ledit hétérocycle étant non substitué ou étant substitué par un groupe choisi parmi un halogène, ledit halogène est préférentiellement un fluor.

Les composés de formule (I) selon l'invention englobent les composés suivants :
N,N,N',N'-Tétraméthyl-10H-phénothiazine-2,8-diamine
N,N-Diméthyl-[8-(4-méthylpipérazin-1-yl)-10H-phénothiazin-2-yl]amine
N,N-Diméthyl-(8-morpholin-4-yl-10H-phénothiazin-2-yl)amine
N,N-Diéthyl-N',N'-diméthyl-10H-phénothiazine-2,8-diamine
N,N-Diéthyl-[8-(4-méthyl-pipérazin-1-yl)-10H-phénothiazin-2-yl]amine
N,N-Diéthyl-(8-morpholin-4-yl-10H-phénothiazin-2-yl)amine
N,N-Diméthyl-N',N'-dipropyl-10H-phénothiazine-2,8-diamine
[8-(4-Méthyl-pipérazin-1-yl)-10H-phénothiazin-2-yl]-dipropylamine
(8-Morpholin-4-yl-10H-phénothiazin-2-yl)-dipropylamine
N,N-Dibutyl-N',N'-diméthyl-10H-phénothiazine-2,8-diamine
N,N-Dibutyl-[8-(4-méthylpipérazin-1 -yl)-10H-phénothiazin-2-yl]amine
N,N-Dibutyl-(8-morpholin-4-yl-10H-phénothiazin-2-yl)amine
N,N-Diméthyl-N'-propyl-10H-phénothiazine-2,8-diamine
3,N,N,N',N'-Pentaméthyl-10H-phénothiazine-2,8-diamine
2-[N-(8-Diméthylamino-10H-phénothiazin-2-yl)-N-(2-hydroxyéthyl)amino]éthanol

### Procédé de préparation des composés de formule (I)

Le demandeur a mis au point un procédé de préparation spécifique pour les composés de formule (I), simple, rapide à mettre en oeuvre, et qui permet notamment l'introduction aisée des divers types de substituants aussi bien sur les fonctions amine (groupes R₂ₐ, R_{2b}, R₈ₐ et R_{ab}) que sur les noyaux aromatiques (groupes R₁, R₃, R₄, R₆, R₇ et R₉) des 2,8-diaminophénothiazines de l'invention.

### Procédé général (étape b)

L'invention a aussi pour objet un procédé pour la préparation d'un composé 2,8-diaminophénothiazine de formule (I') suivante 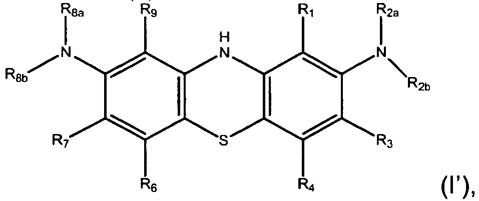 dans laquelle :
(i) les groupes R₁, R₃, R₄, R₆, R₇ et R₉ représentent chacun, indépendamment l'un de l'autre un groupe choisi parmi :
   - un atome d'hydrogène,
   - un halogène,
   - un groupe alkyle ayant de 1 à 12 atomes de carbone non substitué,
   - un groupe alkyle ayant de 1 à 12 atomes carbone substitué par un groupe phényle, ledit groupe phényle étant :
      - soit non substitué,
      - soit substitué par un ou plusieurs groupes choisis parmi un halogène, un hydroxy, un alkyle ayant de 1 à 12 atomes de carbone, et un alcoxy ayant de 1 à 12 atomes de carbone,
(ii) les groupes R₂ₐ, R_{2b}, _{R8a} et R_{8b} représentent chacun, indépendamment l'un de l'autre un groupe choisi parmi :
   - un atome d'hydrogène,
   - un groupe alkyle ou un groupe alcoxyalkyle ayant de 1 à 12 atomes de carbone non substitué,
   - un groupe alkyle, un groupe hydroxyalkyle, ou un groupe alcoxyalkyle ayant de 1 à 12 atomes de carbone substitué par un groupe phényle, ledit groupe phényle étant :
      - soit non substitué,
      - soit substitué, un groupe hydroxy alkyle par un groupe choisi parmi
         - un halogène,
         - un hydroxy,
         - un alkyle ayant de 1 à 12 atomes de carbone,
         - un alcoxy ayant de 1 à 12 atomes de carbone,
         - un alcényle ayant de 2 à 12 atomes de carbone, non substitué ou substitué par un groupe choisi parmi un halogène, un hydroxy, un alkyle ayant de 1 à 12 atomes de carbone, un alcoxy ayant de 1 à 12 atomes de carbone,
         - un alcynyle ayant de 2 à 12 atomes de carbone, non substitué ou substitué par un groupe choisi parmi un halogène, un hydroxy, un alkyle ayant de 1 à 12 atomes de carbone, un alcoxy ayant de 1 à 12 atomes de carbone,
         - un cycloalkyle ayant de 4 à 12 atomes de carbone non substitué ou substitué par un groupe choisi parmi un halogène, un hydroxy, un alkyle ayant de 1 à 12 atomes de carbone, un alcoxy ayant de 1 à 12 atomes de carbone,
         - un cycloalcényle ayant de 4 à 12 atomes de carbone non substitué ou substitué par un groupe choisi parmi un halogène, un hydroxy, un alkyle ayant de 1 à 12 atomes de carbone, un alcoxy ayant de 1 à 12 atomes de carbone,
(iii) ou bien les groupes -NR₂ₐR_{2b} ou -NR₈ₐR_{8b} représentent, indépendamment l'un de l'autre, un hétérocycle ayant de 4 à 12 atomes de carbone, saturé ou insaturé, ledit hétérocycle pouvant comprendre un ou plusieurs hétéroatomes additionnels choisis parmi l'azote, l'oxygène et le soufre, ledit hétérocycle étant non substitué ou étant substitué par un groupe choisi parmi un halogène, un hydroxy et un alkyle ayant de 1 à 12 atomes de carbone,
ledit procédé comprenant une étape (b) au cours de laquelle le composé diphénylamine de formule (II) suivant : 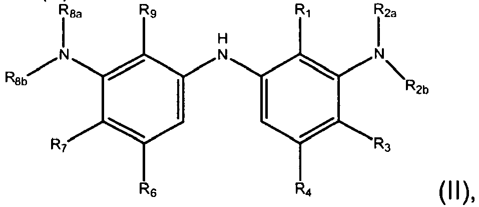 dans lequel les groupes R₁, R₂ₐ, R_{2b}, R₃, R₄, R₅, R₇, R₈ₐ, R_{8b} et R₉ ont la même signification que pour le composé de formule (I),
est soumis à une étape de chauffage choisie-parmi:
(b1) une étape de chauffage à une température allant de 60°C à la température d'ébullition du mélange réactionnel en présence d'iode et de soufre dans un solvant inerte tel qu'un solvant aromatique, un alcane ou un polyhalogène alcane pendant une durée allant de 1 heure à une nuit ;
(b2) une étape de chauffage par micro-ondes pendant une durée allant de 10 secondes à six fois dix minutes ;
   (b2-1) soit d'un mélange du composé de formule (II) avec de l'iode et du soufre, sans solvant ou en présence d'un solvant inerte tel qu'un solvant aromatique, un alcane ou un polyhalogènoalcane ;
   (b2-2) soit d'un mélange du composé de formule (II) avec de l'iode et du soufre, le composé de formule (II), l'iode et le soufre étant adsorbés sur un support tel que la silice, l'alumine ou une zéolite, afin d'obtenir le composé de formule (I).

Pour la réalisation de l'étape b1), on utilise préférentiellement comme produit de départ un mélange comprenant le composé de formule (I), le soufre et l'iode dans une étape de chauffage au reflux, dans un solvant dont le point d'ébullition est approprié pour respecter la température de réaction choisie.

De préférence, à l'étape b1), la température de chauffage est comprise entre 100°C et la température d'ébullition du mélange réactionnel.

A l'étape b1) on peut utiliser notamment un solvant choisi parmi le toluène, ie xylène (ortho-xylène, meta-xylène, para-xylène), le durène, l'isodurène, le dichloro-benzéne (ortho, meta ou para), l'éthylbenzène, le pentaméthylbenzène, l'hexaméthylbenzène, le mésitylène, le cumène ou encore l'hemimellitène. Préférentiellement, on utilise l'ortho dichloro-benzène.

A l'étape b1), le composé de formule (I) et le soufre sont utilisés dans un rapport molaire composé(I)/soufre allant de 0,2/1 à 1/1, par exemple un rapport molaire allant de 0,3/1 à 0,5/1, tel qu'environ 0,4/1.

A l'étape b1), l'iode peut être ajouté sous la forme d'un cristal d'iode.

L'étape b1) est préférentiellement réalisée sous atmosphère inerte et si possible anhydre, par exemple sous atmosphère d'argon, d'azote ou de diazote.

Dans certains modes de réalisation, la durée de l'étape b1) peut aller de 2 heures à 4 heures. Elle peut être d'environ 3 heures.

Après chauffage, la température du milieu réactionnel est réduite jusqu'à température ambiante, puis le composé de formule (I) correspondant est récupéré.

Dans certains modes de réalisation, le composé de formule (I) est récupéré et extrait par l'ajout d'un solvant approprié, choisi parmi les solvants conventionnels appropriés bien connus de l'homme du métier. A titre illustratif, le composé de formule (I) résultant peut être extrait par de l'éther diéthylique, puis éventuellement filtré et concentré sous pression réduite.

Le cas échéant, le liquide huileux comprenant le composé de formule (I) subit une étape finale de purification. L'étape de purification peut consister en une étape de chromatographie, par exemple sur gel de silice, avant élution du composé de formule (1), le cas échéant avec du toluène, afin de fournir une poudre du composé de formule (I).

Pour la réalisation de l'étape alternative b2), le chauffage est réalisé par exposition du composé de formule (II) et du ou des réactifs associés à un rayonnement micro-ondes.

Selon un premier mode de réalisation de l'étape alternative b2), désignée b2-1) ci-dessus, on chauffe par exposition à un rayonnement micro-ondes un mélange comprenant le composé de formule (II), de l'iode et du soufre, afin d'obtenir, puis de récupérer, le composé de formule (I) correspondant. Pour l'essentiel, les conditions de réaction sont identiques à celles décrites pour l'étape b1) ci-dessus, à l'exception des paramètres spécifiques au chauffage proprement dit.

Généralement, on utilise un rayonnement micro-ondes à une puissance allant de 150 à 500 Watts., l'échelle étant compris entre 70 et 700 W.Selon un second mode de réalisation de l'étape alternative b2), désignée b2-1) ci-dessus, on chauffe par exposition au rayonnement micro-ondes un mélange comprenant un composé de formule (II) avec de l'iode et du soufre, l'iode et le soufre étant adsorbés sur un support. Pour l'essentiel, les conditions de réaction sont identiques à celles décrites pour l'étape b1) ci-dessus, à l'exception des paramètres spécifique au chauffage proprement dit, qui sont similaires ou identiques aux paramètres de chauffage décrits pour le premier mode de réalisation désigné b2-1) ci-dessus.

Comme types de supports, pour immobiliser le soufre et l'iode, on peut utiliser par exemple l'alumine, la silice ou une zéolite.

### Etape a) du procédé

De manière générale les divers composés de formule (II) utilisés comme produits de départs pour réaliser le procédé de préparation des composés de formule (I) peuvent être obtenus par toute méthode connue de l'homme du métier.

Toutefois, le demandeur a mis au point des procédés de préparation des composés de formule (II), lesdits procédés consistant en des modes de réalisation spécifiques du procédé général de préparation des composés de formule (I) décrit précédemment.

### Première alternative de l'étape a) du procédé

Ainsi, dans un premier mode de réalisation spécifique, le procédé de préparation d'un composé de formule (I) tel que défini ci-dessus est caractérisé en ce qu'il comprend une étape (a) d'obtention d'un composé de formule (II), qui est préalable à l'étape b), dans laquelle on fait réagir un composé de formule (III) suivante : 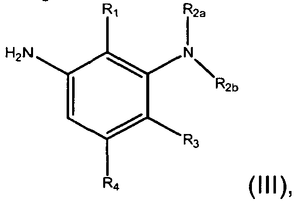 dans laquelle les groupes R₁, R₂ₐ, R_{2b}, R₃ et R₄ ont la même signification que dans la revendication 1 ,
avec un composé de formule (IV) suivante : 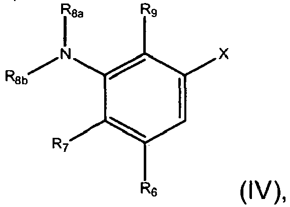 dans laquelle les groupes R₅, R₇, R₈ₐ, R_{8b} et R₉ ont la même signification que dans la revendication 1 et le groupe X représente un halogène ou un groupe sulfonate,
en présence d'un catalyseur de palladium ou de nickel et d'un ligand de catalyseur et d'une base organique ou inorganique,
à une température allant de 80°C à 110°C et pendant une durée allant de 8 à 15 heures pour obtenir le composé de formule (II).

Dans certains modes de réalisation, le groupe X représente un halogène choisi parmi le brome, le chlore ou l'iode.

Dans d'autres modes de réalisation, le groupe X représente un sulfonate choisi parmi un triflate, un mésylate, un tosylate, un nosylate et un nonaflate.

Dans certains modes de réalisation, le catalyseur consiste en un catalyseur à base de platine, par exemple un catalyseur Pd(dba)₂.

Dans certains modes de réalisation, le ligand de catalyseur est choisi parmi la dppa (bis(diphénylphosphino) amine), la dppf (1,1'-bis(diphénylphosphino)ferrocène), le BINAP, le josiphospyphos (6-diphénylphosphino-2-pyridonate), le QUINAP, le Pyphos ou le Qphos, qui sont des ligands de catalyseur bien connus de l'homme du métier. Avantageusement, on utilise dppf.

Dans certains modes de réalisation, la base organique consiste en un carbonate alcalin ou encore du tert-butylate de sodium.

Le mélange de réactifs est avantageusement dissous dans un solvant approprié, qui peut être choisis parmi le toluène, le xylène (ortho-xylène, méta-xylène, para-xylène), le durène, l'isodurène, le dichloro-benzéne (ortho, méta ou para), l'éthylbenzène, le pentaméthylbenzène, l'hexaméthylbenzène, le mesitylènele cumène ou encore l'hemimellitène. Préférentiellement, on utilise l'ortho dichloro-benzène.

Avantageusement, le rapport molaire des composés (III)/(IV) dans le mélange réactionnel est compris entre 0,5/1 et 1/0,5, préférentiellement entre 0,8/1 et 1,2/1, et est de manière tout à fait préférée d'environ 1.

Après chauffage, la température du milieu réactionnel est réduite jusqu'à température ambiante, puis le composé de formule (II) correspondant est récupéré.

Dans certains modes de réalisation, le composé de formule (II) est récupéré et extrait par l'ajout d'un solvant approprié, choisi parmi les solvants conventionnels appropriés bien connus de l'homme du métier. Le composé de formule (II) résultant, après extraction, peut être filtré et concentré sous pression réduite.

Dans d'autres modes de réalisation, le composé de formule (II), le composé de formule (II) résultant est purifié par flash chromatographie, par exemple en utilisant un gradient d'un solvant approprié. Illustrativement, on peut utiliser un gradient allant de 5% (v/v) à 12% (v/v) d'acétate d'éthyle dans l'hexane, comme illustré dans les exemples.

### Seconde alternative de l'étape a) du procédé

Selon un second mode de réalisation spécifique, le procédé de préparation d'un composé de formule (I) tel que défini ci-dessus est caractérisé en ce qu'il comprend une étape (a) d'obtention d'un composé de formule (II), qui est préalable à l'étape b), dans laquelle on fait réagir un composé de formule (III) suivante : 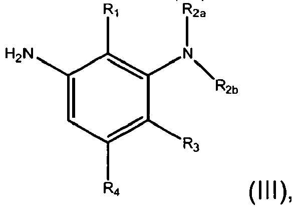 dans laquelle les groupes R₁, R₂ₐ, R_{2b}, R₃ et R₄ ont la même signification que dans la revendication 1,
avec un composé de formule (IV) suivante : 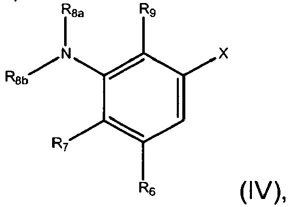 dans laquelle les groupes R₅, R₇, R₈ₐ, R_{8b} et R₉ ont la même signification que dans la revendication 1 et le groupe X représente un halogène ou un groupe sulfonate, en présence d'un sel ou oxyde de cuivre et d'une base organique à une température allant de 40°C à la température d'ébullition du milieu réactionnel pendant une durée allant de 2 heures à une nuit pour obtenir le composé de formule (II).

Dans certains modes de réalisation, le groupe X représente un halogène choisi parmi le brome, le chlore ou l'iode.

Dans d'autres modes de réalisation, le groupe X représente un sulfonate choisi parmi un triflate, un mésylate, un tosylate, un nosylate et un nonaflate.

Dans certains modes de réalisation, la base organique consiste en un carbonate alcalin tel que le carbonate de sodium, le carbonate de potassium et le carbonate de césium.

Dans certains modes de réalisation, le sel de cuivre est choisi parmi les halogénures de cuivre.

### Autres alternatives de préparation des composés de formule (I)

Les composés de formule (I) peuvent également être préparés à partir de composés de formule (I) dans lesquels R₂ₐ, R_{2b}, R₈ₐ ou R_{8b} représentent un atome d'hydrogène, par alkylation, amination réductrice ou par acylation suivie d'une réduction.

Lorsqu'on opère par une alkylation, l'amine est condensée sur un halogénure d'alkyle ou un sulfonate en présence d'une base inorganique telle qu'un carbonate ou un bicarbonate métallique ou d'une base organique telle qu'une amine tertiaire.

Lorsqu'on opère par une amination réductrice, l'amine est condensée sur une cétone ou un aldéhyde en présence d'un agent réducteur tel que le dihydrogène ou un donneur de dihydrogène (formiate, cyclohexène par exemple) en présence ou non d'un métal de transition tel que le palladium. Il est également possible d'utiliser comme agent réducteur un hydrure tel que le cyanoborohydrure de sodium, le triacétoxyborohydrure de sodium ou le borohydrure de sodium.

Lorsqu'on procède à une acylation suivie d'une réduction, l'acylation peut être réalisée par action d'un anhydride symétrique ou mixte, un chlorure d'acide ou un acide en présence d'un agent de couplage tel qu'un carbodiimide, le carbonyldiimidazole ou un autre agent de couplage peptidique en présence éventuellement d'un catalyseur tel que la 4-diméthylaminopyridine, l'hydroxybenzotriazole (HOBT) ou le HOAT et d'une base organique ou inorganique. La réduction peut ensuite être réalisée à l'aide d'hydrure double de lithium et d'aluminium.

Ainsi, selon encore un autre mode de réalisation du procédé de préparation d'un composé de formule (I) selon l'invention, ledit procédé est caractérisé en ce que un composé de formule (I') dans laquelle au moins un des groupes choisis parmi R₁, R₂ₐ, R_{2b}, R₃, R₄, R₅, R₇, R₈ₐ, R_{8b} et R₉ ne représente pas un atome d'hydrogène est préparé à partir d'un composé de formule (I") dans laquelle chacun des groupes choisis parmi R₁, R₂ₐ, R_{2b}, R₃, R₄, R₅, R₇, R₈ₐ, R_{8b} et R₉ représente un atome d'hydrogène selon les étapes suivantes :
c) une étape réactionnelle choisie parmi une étape d'alkylation, une étape d'amination réductrice ou une étape d'acylation ; et
d) une étape de réduction.

Les composés de formule (I) dans lesquels R₁, R₃, R₄, R₆, R₇ ou R₉ est différent de hydrogène peuvent être préparés à partir de composés de formule (I) dans lesquels R₁, R₃, R₄, R₆, R₇ ou R₉ représente un atome d'hydrogène par les méthodes de fonctionnalisation des noyaux aromatiques connues de l'homme de l'art incluant d'une manière non limitative les halogénations, les métallations dirigées, les réactions de Friedel et les réarrangements.

Ainsi, selon encore un autre mode de réalisation du procédé de préparation d'un composé de formule (I) selon l'invention, ledit procédé est caractérisé en ce que on obtient un composé de formule (I') dans laquelle au moins un des groupes choisis parmi R₁, R₂ₐ, R_{2b}, R₃, R₄, R₅, R₇, R₈ₐ, R_{8b} et R₉ ne représente pas un atome d'hydrogène est préparé à partir d'un composé de formule (I") dans laquelle chacun des groupes choisis parmi R₁, R₂ₐ, R_{2b}, R₃, R₄, R₅, R₇, R₈ₐ, R_{8b} et R₉ représente un atome d'hydrogène selon une étape choisie parmi :
- une réaction d'halogénation,
- une réaction de métallation dirigée,
- une réaction de Friedel, et Craft
- une réaction de réarrangement, par exemple une réaction de Fries qui transfère un groupement acyle d'un oxygène ou d'un azote sur un aromatique.

Les anilines de formule (III) et les dérivés aromatiques de formule (IV) sont accessibles au public, par exemple commercialement accessible auprès des fournisseurs de produits chimiques.

Alternativement, les composés de formules (III), comme les composés de formule (IV), peuvent être préparés par application ou adaptation de méthodes décrites et connues de l'homme du métier.

Dans les réactions décrites précédemment, il peut s'avérer nécessaire de protéger certains groupes fonctionnels afin d'éviter des réactions secondaires non désirées. Ces protections peuvent être réalisées selon les méthodes décrites par T.W. Greene et P. G. M. Wuts dans Protective Groups in Organic Chemistry, John Wiley and Sons, 1991 ou par J. F. W. McOmie dans Protective Groups in Organic Chemistry, Plenum Press, 1973.

Les composés de formule (I) peuvent également être préparés par amination de phénothiazines portant un halogène ou un sulfonate en position 2 ou 8. Cette réaction s'effectue de préférence par couplage en utilisant un métal de transition tel que le palladium, le nickel ou le cuivre.

Ces transformations de groupes fonctionnels peuvent être également adaptées des méthodes décrites par R.C. Larock dans Comprehensive Organic Transformations, VCH publishers, 1989.

### Application industrielle des composés de formule (I)

Comme cela a déjà été indiqué précédemment dans la présente description, les composés de formule (I) possèdent des propriétés biocides d'intérêt industriel, après photoactivation.

Contrairement aux composés de la famille des 3,7-diaminophénothiazines, les composés de formule (I) consistent en des bases mésomériques stables, qui ne présentent pas le phénomène de délocalisation de charge caractéristique du bleu de méthylène et des DAP 3-7

Les composés de formule (I) ne présentent pas non plus une structure ionique de type phénothiazinium, caractéristique des séries 3-7

Comme déjà précisé précédemment dans la présente description, les composés de formule (I) existent à la fois sous la forme de bases et de solvates.

De manière générale, on a montré selon l'invention que les composés de formule (I) présentent au moins trois caractéristiques d'activité qui les distinguent des produits connus, y compris les diaminophénothiazines connues, respectivement :
- les composés de formule (I) consistent en des agents photoactivables par de la lumière blanche. Ainsi, pour photoactiver les composés de formule (I), il n'est pas nécessaire d'exposer ces composés à de la lumière à des longueurs d'ondes du rayonnement ultraviolet. Lorsqu'ils sont exposés à de la lumière blanche, c'est-à-dire à un ensemble de longueurs d'ondes allant d'environ 450 nanomètres à environ 750 nanomètres, les composés de formule (I), par exemple la DAP 2-8 tétraméthyl (N,N,N',N'-tétraméthyl-10H-phénotiazine-2,8-diamine), passe d'une couleur jaune à une couleur verte, plus précisément à une couleur verte nickel.
- les composés de formule (I), après photoactivation avec de la lumière blanche, présentent un spectre d'activité biocide, à l'encontre notamment des bactéries et des parasites, très différent du spectre d'activité biocide du bleu de méthylène. En particulier, les composés de formule (I) possèdent un niveau et un spectre d'activité biocide absolument différent du bleu de méthylène, ces premiers étant actifs dès la concentration de 0,05 pour mille (poids/poids) dans de l'eau distillée. De plus, les composés de formule (I) sont photoactivés par une exposition à une intensité lumineuse plus faible que l'intensité lumineuse qui est nécessaire pour photoactiver les composés 3,7-diaminophénothiazine connus (tests normés - NF EN 1040).
- Certains composés de formule (I), comme par exemple la N,N,N',N'-tétraméthyl-10H-phénotiazine-2,8-diamine, ou la N,N-Dibutyl-(8-morpholin-4-yl-10H-phénothiazin-2-yl)amine présentent aussi des propriétés d'agents floculants. En particulier, on a montré selon l'invention que les composés de formule (I) se comportent comme des agents de floculation des protéines, aussi bien animales que bactériennes. On a aussi montré que l'effet de floculation qui est obtenu est proportionnel à la quantité de composé de formule (I) qui est ajouté. Dans la pratique, le composé floculant de formule (I) se concentre dans le précipité (floc). L'effet d'agent floculant d'un composé de formule (I) est détectable pour une concentration de composé de formule (I) aussi faible que 0,1 pour mille (poids/poids), voire moins (seuil vers 0,05 pour mille). Il existe un rapport structure activité du pouvoir floculant, corrélé avec la longueur des chaines latérales et le nombre de groupements alcanes et/ou hétérocycliques (par exemple pipérazine, morpholine). De plus, certains composés de formule (1) floculent spontanément à la lumière dans de l'eau distillée.

La combinaison des effets techniques qui peuvent être induits par les composés de formule (1) peuvent être mis à profit dans toutes les applications industrielles dans lesquelles on recherche un effet désinfectant.

Tout particulièrement, les composés de formule (I) sont utiles dans le domaine du traitement des eaux, qu'il s'agisse du traitement des eaux en vue de la production d'eau potable, ou bien du traitement des eaux usées préalable à leur rejet dans l'environnement naturel.

En particulier la DAP-2,8 tétraméthyl à des concentrations de 100 mg/l à 1000 mg/l ne présente aucune activité irritant corrosif chez le lapin albinos (classification non irritante pour la peau et les yeux selon OCDE 404 et 405), ce qui est remarquable pour un agent biocide.

Comme cela a été décrit dans la partie de la description relative à l'art antérieur, l'étape de floculation/décantation et l'étape de désinfection sont aujourd'hui des étapes distinctes, dans les procédés courants de traitement des fluides, y compris des effluents industriels ou domestiques. Dans les procédés courants, l'étape de floculation/décantation aboutit notamment à la production de boues très riches en des types très variés de micro-organismes. C'est la raison pour laquelle, selon ces procédés de traitement conventionnels, une étape ultérieure de désinfection des fluides est requise afin d'éliminer ces mico-organismes, parfois pathogènes pour l'homme et les animaux, avant le rejet de l'effluent traité dans l'environnement. Quant aux boues obtenues, elles sont un réservoir d'organismes vivants de toute taille, et de polluants, potentiellement dangereux.

Ainsi, grâce aux composés 2,8-diaminophénothiazine de formule (I), il est désormais possible de réaliser simultanément (i) la décantation des polluants après floculation et (ii) la désinfection du fluide en cours de traitement.

Désormais, grâce à l'utilisation des composés de formule (I), il peut être mis au point des procédés de traitement des fluides, notamment des effluents industriels ou domestiques, comprenant une étape unique de floculation/désinfection/décantation.

Un autre avantage des composés 2,8-diaminophénothiazine de formule (I) réside dans le fait que l'induction de leur activité biocide ne requiert pas obligatoirement de dispositif de photoactivation spécifique, comme par exemple des dispositifs générateurs d'un rayonnement ultraviolet.

Par exemple, pour permettre aux composés 2,8-diaminophénothiazine de formule (I) d'exercer leurs effets combinés d'agents floculants et d'agents biocides, il suffit que l'étape de floculation/désinfection soit réalisée dans un bassin ou une cuve de traitement exposé à la lumière naturelle.

Ainsi, la présente invention a aussi pour objet l'utilisation d'un composé de formule (I) tel que défini dans la présente description, en tant qu'agent biocide. L'utilisation de ces composés comme agents biocides peut être réalisée dans le domaine de la désinfection, l'épuration, la stérilisation ou la purification des fluides.

Dans certains modes de réalisation, lesdits fluides sont choisis parmi les effluents industriels et les effluents domestiques.

L'invention concerne aussi une composition pour la désinfection, l'épuration, la stérilisation ou la purification des fluides comprenant un composé de formule (I) tel que défini dans la présente description.

Dans certains modes de réalisation, ladite composition se présente sous la forme d'un support sur lequel, ou dans lequel, est immobilisé le composé de formule (I).

Ainsi, les produits peuvent être incorporés et réticulés chimiquement dans un plastique inerte, par exemple de type polyuréthanne, de dureté et de formes variable (plaques, billes, poudre, mousse, lamelles, pales de ventilateurs, hélices etc...). Les produits sont incorporés avant polymérisation sous forme de bases hydrophobes. Après réaction, les plaques obtenues sont de couleur variées. La concentration finale en composé(s) de formule (I) est de 0,1 à 5 % en poids, par rapport au poids total de la composition finale. Ces plaques sont utilisables comme support de photoactivation biocide en lumière solaire ou artificielle. Un écoulement contrôlé des eaux ou de gaz sous lumière naturelle ou électrique, induit la génération de molécules et de radicaux biocides, permettant une réduction significative de la charge microbienne, peu onéreuse, et rapide des effluents, qui peuvent êtres disposés entre deux plaques. Ce type de procédé est particulièrement adapté aux circuits fermés d'eau potable, telles les habitations ne disposant pas d'adduction correcte mais disposant d'eaux de pluie ou de sources, ruisseau, réservoirs, etc, de communautés humaines isolées, de bateaux, d'avions, de trains, d'unités mobiles en environnements hostiles, etc. La réaction étant de type catalytique, il n'y a aucun relargage du produit lors de la désinfection.

Le procédé est également applicable en traitement des gaz et des circuits de distribution (systèmes air conditionnés, tours aéroréfrigérantes, gaz médicaux ou en usage industriel. Le gaz peut être envoyé dans des systèmes appropriés, illuminés en lumière blanche. L'incorporation des DAP 2-8 peut se faire alors sur un support inerte type plastique ou céramique, ou un support inerte poreux, par exemple du non-tissé, des dérivés cellulosiques, ou toutes autres structures à cellules ouvertes.

L'immobilisation du produit sur le support peut se faire de manière covalente ou non covalente. Dans l'option covalente, le produit à immobiliser comporte un site réactif qui lui permet d'être attaché au support lors de la réaction de polymérisation. Par exemple, le produit peut comporter un alcool dans l'un des substituants R₁, R₃, R₄, R₆, R₇, R₉, R₂ₐ, R_{2b}, R₈ₐ ou R_{8b}, qui réagira avec un isocyanate lors de la préparation d'un support en polyuréthane. Dans l'option non covalente, le produit peut être mélangé à l'un au moins des monomères avant la réaction de polymérisation. Il est également possible d'appliquer le produit sur le support, éventuellement en le diluant dans un solvant tel que de l'eau ou un solvant organique. L'application pouvant être réalisé par trempage du support, pulvérisation du produit dilué ou à l'aide de pinceau ou rouleau.

L'invention est en outre illustrée par ses modes de réalisation décrits dans les exemples.

### EXEMPLES

### Exemple 1 : N,N,N',N'-tétraméthyl-10H-phénothiazine-2,8-diamine

### Etape A : bis-(3-diméthylaminophényl)amine

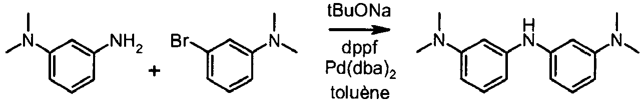

Dans un ballon, on introduit sous atmosphère de diazote, 1 équivalent de 3-bromo-N,N-diméthylaniline (1g), 1.5 équivalent de tert-butylate de sodium (0,721 g), 0,1 équivalent de Pd(dba)₂ (0,288 g), 0,12 équivalent de dppf (0,555 g) et 20 mL de toluène. Le ballon est équipé d'un septum en PTFE et 1,2 équivalent de N,N-diméthylbenzène-1,3-diamine (0,817g) est ajouté via une seringue. Le milieu réactionnel est chauffé à 90°C pendant 12 h puis refroidit à température ambiante et concentré sous pression réduite. Le produit est purifié par flash chromatographie en utilisant un gradient de 5% à 12% d'acétate d'éthyle dans l'hexane (Rf: 0,33) pour donner 0,963 g de produit attendu avec un rendement de 75%.

RMN ¹H : (CDCl₃) 7.13(t, 2H) 6.5(d, 2H), 6.49 (d, 2H), 6.38-6.36 (m, 2H), 5.68 (brs, 1H), 2.95 (s, 12H).
MS (M+1): 256

### Étape B : N,N,N',N'-tétraméthyl-10H-phénothiazine-2,8-diamine

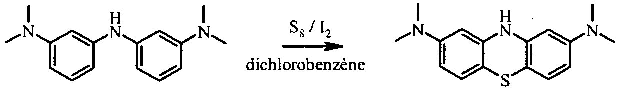

Un mélange de bis-(3-diméthylaminophényl)amine (0.5 g, 1.9 mmol), de soufre (0,15 g, 4,6 mmol) et un cristal d'iode dans 4 mL d'o-dichlorobenzene est chauffé au reflux sous atmosphère de diazote pendant 3 h. Après retour à température ambiante, le mélange est extrait par de l'éther diéthylique (15 mL), filtré et concentré sous pression réduite.

L'huile correspondante est chromatographiée sur gel de silice avec du toluène comme éluant pour donner une poudre brune (140 mg).

### Mode opératoire général du couplage.

Sous atmosphère inerte (argon) sur l'aromatique bromé IV (1 équiv) en solution dans le toluène (4 mL par mmol) sont successivement ajoutés l'aniline III (1.2 équiv) puis Pd(dba)2 (0.05 équiv), dppf (0.1 équiv) et tBuONa (1.5 équiv). Le mélange réactionnel est dégazé et placé sous atmosphère d'argon puis porté au reflux du toluène de 8 à 14 heures (jusqu'à complète disparition de IV). Le mélange réactionnel est ensuite ramené à température ambiante, partitionné entre eau et acétate d'éthyle puis extrait à l'acétate d'éthyle (2 fois). Les phases organiques sont regroupées, séchées sur sulfate de sodium et les solvants évaporés. Le mélange brut est purifié par chromatographie sur gel de silice pour conduire à la diarylamine II avec des rendements supérieurs à 60%. Éluants : cyclohexane - acétate d'éthyle 80 / 20 à 60 / 40 sauf pour les composés avec une N-méthylpipérazine pour lesquels on utilise : dichlorométhane - méthanol 90 /10 à 80 / 20.

### Mode opératoire général de formation de phénothiazines I.

A une solution de diarylamine II (1 équiv) dans le 1,2-dichlorobenzène (1 mL pour 100 mg de substrat) maintenue sous atomsphère d'argon, sont ajoutés du soufre (0.3 équiv) et un cristal de diiode. Le mélange réactionnel est porté au reflux et maintenu sous agitation à cette température pendant 4 à 5 heures. Après retour à température ambiante, sur le mélange réactionnel est ajouté du dichlorométhane et une solution saturée de thiosulfate de sodium. Après décantation, la phase organique est séparée de la phase aqueuse puis séchée sur sulfate de sodium. Après évaporation du dichlorométhane, le brut réactionnel en solution dans le 1,2-dichlorobenzène est directement purifié par chromatographie sur gel de silice pour conduire à la phénothiazine I avec des rendements variables suivant les substrats [la colonne de chromatographie est protégée de la lumière par une feuille d'aluminium]. Dans certains cas, le substrat issu de la purification est solubilisé dans du diéthyléther à chaud sur lequel est ajouté du noir de carbone. Le mélange est filtré à travers un lit de célite et le solvant est évaporé pour donner, en général, la phénothiazine I sous la forme d'un solide brun clair.
Éluants : cyclohexane - acétate d'éthyle 80 / 20 à 60 / 40 sauf pour les composés avec une N-méthylpipérazine pour lesquels on utilise : dichlorométhane - méthanol 90 /10 à 80/20.

### Mode opératoire général de formation de chlorhydrates de phénothiazines I.

Sur la phénothiazine sous forme de base en solution dans l'éthanol absolu (3 à 12 mL pour 0.3 mmol suivant les substrats) est ajoutée, à une température voisine de 0 °C, une solution d'HCl dans le diéthyléther (2 N, 4 équiv, 1.2 mmol). Le mélange réactionnel est agité 15 minutes à une température voisine de 0 °C, puis les solvants sont évaporés sous pression réduite. Le résidu est trituré dans le diéthyléther anhydre jusqu'à obtention d'un solide. Le solide est séparé par filtration sur fritté, lavé à plusieurs reprises par du diéthyléther anhydre puis séché à l'étuve sous vide. Les chlorhydrates de phénothiazines sont isolés avec des rendements de l'ordre de 90%.

En utilisant ces modes opératoires généraux, on prépare les produits suivants caractérisés par leur spectre de résonnance magnétique nucléaire à 270MHz réalisé sur le chlorhydrate en solution dans le méthanol deutéré :

| Exemple | Structure | RMN |
|---|---|---|
| 2 | 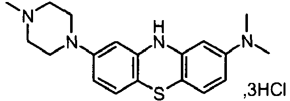 | 2,90 (3 H, s), 3,30 (10 H, br m), 3,55 (2 H, m), 3,80 (2 H, br s), 7,05 (6 H, m) |
| 3 | 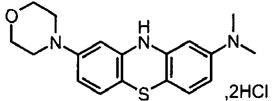 | 3,23 (6 H, m), 3,61 (4 H, m), 4,09 (4 H, m), 7,03 (6 H, m) |
| 4 | 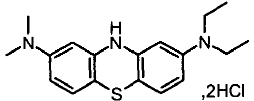 | 1,15 (6 H, m), 3,15 (6 H, br s), 3,45 (4 H, br s), 7,05 (6 H, m) |
| 5 | 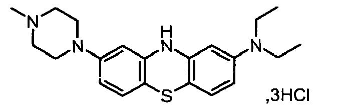 | 1,17 (6 H, t, J = 7,0 Hz), 2,95 (3 H, s), 3,05-3,90 (12 H, br m), 6,45 (2 H, br s), 7,05 (4H, br m) |
| 6 | 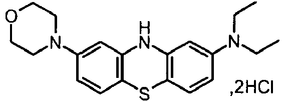 | 1,17 (6 H, t, J = 7,0 Hz),3,48 (8 H, m), 4,05 (4 H, m), 7,05 (6 H, m) |
| 7 | 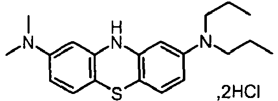 | 0,95 (6 H, br t, J = 7,3 Hz), 1,49 (4 H, m), 3,29 (6 H, br s), 3,50 (4 H, m), 7,05 (6 H, m) |
| 8 | 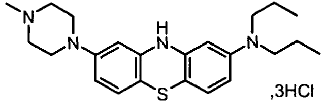 | 0,95 (6 H, m), 1,52 (4 H, m), 2,90-3,80 (15 H, m), 6,50-7,05 (6 H,m) |
| 9 | 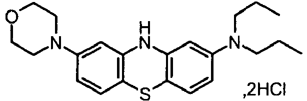 | 0,95 (6 H, br t, J = 6,8 Hz), 1,55 (4 H, m), 3,50 (4 H, m), 3,60 (4 H, m), 4,08 (4 H, m), 7,00 (6 H, m) |
| 10 | 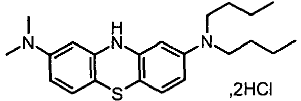 | 0,91 (6 H, m), 1,15 - 1,55 (8 H, m), 3,39 (6 H, br s), 3,55 (4 H, m), 7,05 (6 H, m) |
| 11 | 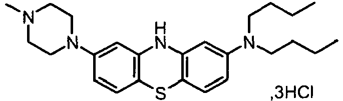 | 0,95 (6 H, m), 1,35 (6 H, m), 1,59 (2 H, m), 2,90-3,90 (15 H, m), 6,50-7,05 (6 H, m) |
| 12 | 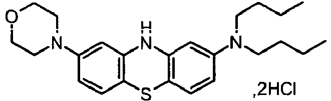 | 0,90 (6 H, m), 1,35 (8 H, m), 3,01 (4 H, m), 3,50 (4 H, m), 3,82 (4 H, m), 4,06 (2 H, m), 6,91 (6 H, m), |
| 13 | 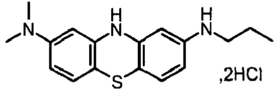 | 1,04 (3 H, br t, J = 7,4 Hz), 1,75 (2 H, m), 3,23-3,32 (8 H, m), 6,90 (6 H, m) |
| 14 | 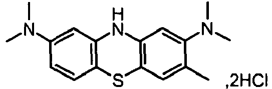 | 2,35 (s, 3 H), 3,25-3,29 (m, 6 H), 6,93-7,06 (m, 5 H) |

Par ailleurs, certains produits ont également été caractérisés par résonnance magnétique nucléaire du carbone 13 réalisé sur le chlorhydrate à 62,5MHz dans le méthanol deutéré :

| Exemple | Structure | RMN |
|---|---|---|
| 13 | 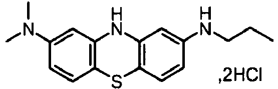 | 8,87, 18,23, 44,77, 52,51, 105,20, 107,35, 112,51, 115,20, 118,10, 118,66, 126,49, 126,65, 134,10, 141, 52, 141, 94, 142,09 |
| 14 | 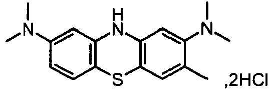 | 16,35, 46,98, 47,19, 106,57, 120,59, 121,10, 124,50, 124,96, 128,86, 130,69, 141,46, 142,27, 144,05, 144,26 |

La structure de certains exemple a également été confortée par une analyse par spectrométrie de masse à haute résolution

| Exemple | analyse |
|---|---|
| 10 | calcd : 369,2238; found : 369,2272 |
| 11 | calcd : 424,2661; found : 424,2694 |
| 13 | calcd : 299,1456; found : 299,1468 |

Le point de fusion de quelques bases est donné à titre indicatif

| Exemple | point de fusion |
|---|---|
| 8 | 122-124°C |
| 11 | 150-152°C |
| 13 | 158-160°C |

### Exemple 15 : 2-[N-(8-diméthylamino-10H-phénothiazin-2-yl)-N-(2-hydroxyéthyl)amino]éthanol et dichlorhydrate de 2-[N-(8-diméthylamino-10H-phénothiazin-2-yl)-N-(2-hydroxyéthyl)amino]éthanol

### Etape 15.1 : (3-bromophenyl)-bis-[2-(tert-butyldiméthylsilanyloxy)éthyl]amine

Sous atmosphère d'argon, un mélange de 2-[N-(3-bromophenyl)-N-(2-hydroxyéthyl)amino]éthanol (1,15 g, 4,42 mmol), de chlorure de tert-butyldiméthylsilyle (3 équiv, 2,0 g, 13,27 mmol) et d'imidazole (6 équiv, 1,8 g, 26,5 mmol) dans du dichlorométhane (40 mL) est agité à température ambiante une nuit. Après partition du mélange réactionnel entre dichlorométhane et eau, extractions avec dichlorométhane (deux fois). Les phases organiques sont regroupées, séchées sur sulfate de sodium, filtrées et le solvant éliminé. Après purification par chromatographie sur gel de silice, la (3-bromophenyl)-bis-[2-(tert-butyldiméthylsilanyloxy)éthyl]amine est obtenue sous la forme d'une huile incolore.
1H NMR (270 MHz, CDCl3) : δ (ppm) : 0,03 (12 H, m), 0,88 (18 H, m), 3,47 (4 H, t, J = 6,2 Hz), 3,74 (4 H, t, J = 6,2 Hz), 6,61 (1 H, br d, J = 8,1 Hz), 6,75 (1 H, br d, J = 8,1 Hz),7,6 Hz), 6,84 (1 H, br s), 7,01 (1 H, br t, J = 8,1 Hz)

### Etape 15.2 : [3-(3-diméthylaminophenylamino)phenyl]-bis-[2-(tert-butyldiméthylsilanyloxy)éthyl]amine

En opérant comme décrit dans le mode opératoire général du couplage, mais en partant de 1,85 g (3,8 mmol) de (3-bromo-phenyl)-bis-[2-(tert-butyldiméthylsilanyloxy)éthyl]amine et de 1,15 g (4,42 mmol) de N,N-diméthylbenzène-1,3-diamine, on obtient la [3-(3-diméthylaminophenylamino)phenyl]-bis-[2-(tert-butyldiméthylsilanyloxy)éthyl]amine sous la forme d'une huile jaune pâle.
¹H NMR (270 MHz, CDCl₃) : δ (ppm) : 0,02 (12 H, m), 0,88 (18 H, br s), 2,92 (6 H, br s), 3,47 (4 H, t, J = 6,2 Hz), 3,74 (4 H, t, J = 6,2 Hz), 5,61 (1 H, br s), 6,28 (2 H, m), 6,40 (4 H, m), 7,10 (2 H, m)

### Etape 15.3 : N,N-bis-(tert-butyldiméthylsilanoxy)éthyl-N',N'-diméthyl-10H-phénothiazine-2,8-diamine

En opérant comme décrit dans le mode opératoire général de formation des phénothiazine, mais en partant de 1,85 g (3,4 mmol) de [3-(3-diméthylaminophenylamino)phenyl]-bis-[2-(tert-butyldiméthylsilanyloxy)éthyl]amine et de 260 mg (0,1 mmol) de soufre, on obtient la N,N-bis-(tert-butyldiméthylsilanoxy)éthyl-N',N'-diméthyl-10H-phénothiazine-2,8-diamine isolé sous la forme d'un solide brun.

### Etape 15.4 : 2-[N-(8-diméthylamino-10H-phénothiazin-2-yl)-N-(2-hydroxyéthyl)amino]éthanol

A une solution de N,N-bis-(tert-butyldiméthylsilanoxy)éthyl-N',N'-diméthyl-10H-phénothiazine-2,8-diamine (1,08 g, 1,88 mmol) dans le tétrahydrofurane (20 mL) est ajoutée une solution de fluorure de tétrabutylammonium 1 M dans le tétrahydrofurane (2,5 équiv, 4,7 mL, 4,7 mmol). Le mélange est agité une heure à température ambiante (complète disparition du produit de départ d'après CCM). La suspension formée est séparée par filtration. Le précipité est lavé avec du tétrahydrofurane, puis solubilisé dans un mélange eau-acétate d'éthyle. La phase organique est ensuite lavée à plusieurs reprises par de l'eau, séchée sur sulfate de sodium. Après élimination du solvant, un solide léger brun foncé est obtenu.

### Etape 15.5 : dichlorhydrate de 2-[N-(8-diméthylamino-10H-phénothiazin-2-yl)-N-(2-hydroxyéthyl)amino]éthanol

En opérant comme décrit dans le mode opératoire général de formation des chlorhydrate, mais en partant de 2-[N-(8-diméthylamino-10H-phénothiazin-2-yl)-N-(2-hydroxyéthyl)amino]éthanol, on obtient le dichlorhydrate de 2-[N-(8-diméthylamino-10H-phénothiazin-2-yl)-N-(2-hydroxyéthyl)amino]éthanol
1H NMR (270 MHz, CDCl3) : δ (ppm) : 3,29 (6 H, m), 3,60 (8 H, m), 7,05 (6 H, m)

### Propriétés de photoactivation des composés de formule (I)

Les tests biocides se font selon la directive NF EN 1040. Les essais sont effectués parallèlement en obscurité et en lumière blanche, correspondant à en ensoleillement solaire moyen (400 KJ/M2) avec une lampe à incandescence ou halogène banale. Certains tests peuvent être effectués jusqu'à 1,3 mW/Cm²

Les phénothiazine-2,8-diamines (DAP-2,8) de l'invention, sous forme de dichlorhydrates, sont mise en solution (100 mg/l) dans l'eau bi distillée ou de type PPI. Par exemple, la couleur de la solution de N,N,N',N'-tétraméthyl-10H-phénotiazine-2,8-diamine passe du jaune au vert nickel en moins de 15 mn. Le virage est d'autant plus rapide que la solution est illuminée en lumière blanche. Les tests sont effectués en flacons de verre stérile selon les normes des essais de désinfection. L'illumination se fait en lumière solaire ou artificielle, en comparaison avec des essais à l'obscurité (verrerie enveloppée de papier d'aluminium).

### Propriétés biocides des composés de formule (I)

### 1) En poudre

Les tests de pouvoir biocide sont effectués selon les normes EN, par exemple EN 1040, EN 1275, sur des souches microbiologiques de référence, avec code ATCC et CIP, et validés par un laboratoire agrée. Exemple du spectre d'action de la N,N,N',N'-Tétraméthyl-10H-phénothiazine-2,8-diamine

| Souche | Concentration (eau distillée) | Temps contact | Réduction titre infectieux (log) |
|---|---|---|---|
| *Legionella pneumophila subsp pneumophila* CIP 103 854 | 100 mg/l | 15 à 30mn | >5,5 |
| *Pseudomonas aeruginosa* CIP 103 467 | 50 à 100 mg/l | 15 à 30 mn | >5,5 |
| *Escherichia coli* CIP 54 127 | 50 à 100 mg/l | 15 à 30 mn | >5,5 |
| *Candida albicans* IP 48.72 | 100 mg/l | 30 mn | 3,2 |

Ces tests valident comme agents biocides au sens de la pharmacopée européenne si la réduction du titre infectieux est supérieur à 5.

Les essais sont menés en lumière blanche contre un témoin obscur. Dans tous les cas, la lumière blanche accélère le phénomène biocide, par exemple l'activité biocide en lumière demande 15 mn de temps de contact, contre 30 mn à l'obscurité.

Il est noter que les DAP 2,8 peuvent présenter un effet de zone, c'est à dire qu'il existe une zone de concentration plus efficace que les concentrations supérieures. Par exemple, la N,N,N',N'-Tétraméthyl-10H-phénothiazine-2,8-diamine est plus active, en lumière à 30 mn de contact, sur *Candida albicans* IP 48.72 à 100 mg/l (3,2 log de réduction du titre infectieux) qu'a 250 ou 500 mg/l (<2,8 log de réduction du titre infectieux

### Propriétés de floculation des composés de formule (I)

En présence de protéines ou d'extrait de levure, les DAP-2,8 précipitent spontanément les milieux de culture (albumine bovine, extrait de levure), en formant un floc de couleur brune. Plus la solution est concentrée en protéines, plus la floculation est importante, Les DAP-2,8 se concentrent dans ce floc. Par exemple dans une solution d'extrait de levure à 10 g/l, plus de 70 % de la quantité initiale de DAP est retrouvée dans le culot. Le pouvoir floculant est d'autant plus important selon :
- Que les chaines latérales en 2-8 présentent des hétérocycles, par exemple le groupement morpholine ou pipérazine.
- Et/ou que les chaines latérales présentent des groupements alkyls de PM croissant (les dérivés butyl sont plus efficaces que les propyls, eux-mêmes plus efficaces que les groupements propyl, puis ethyl et enfin methyls)

Le pouvoir floculant des DAP 2-8 apparaît selon les structures dès 0,05 pour mille. De même, le pouvoir floculant apparaît par diffusion en milieu gélosé, lorsque des disques imprégnés de DAP 2-8 sont déposés sur une culture bactérienne en milieu solide (par exemple, le milieu de Müller Hinton). Il est noté des anneaux de floculation autour des disques imprégnés.

### 2) Support biocide

Les produits sous formes de bases sont incorporés dans des plastiques inertes de type polyuréthanne, ou tous autre support inerte hydrophobe. Les produits sont alors utilisables pour la désinfection/purification/désodorisation de fluides, liquides ou gazeux. L'illumination des plaques induit la production in situ d'oxygène singulet et des radicaux biocides à partir de l'oxygène ambiant ou dissous. La réaction est de type catalytique. La forme de ces plastiques peut être variable selon l'utilisation : lamelles assemblées, plaques déclives, poudre, billes, hélices, pales, etc... Il est également possible d'incorporer les DAP 2-8, par exemple sous forme de solvates, dans des support poreux hydrophiles.

Exemple de préparation : 0,018 g de DAP-2,8 tétraméthyl sous forme de base sont mis en suspension dans 11,075 g de polyol. Après agitation à température ambiante, l'ensemble du polyol prend une teinte jaune limpide. Le rajout de 0,016 g, puis de 0,086 g g de DAP-2,8 tétraméthyl, suivi d'un étuvage à 70°C permet la dissolution complète. La concentration finale est de 0,120 g de DAP-2,8 tétraméthyl dans 11,075 g de polyols

La polymérisation avec un isocyanate dans une proportion de 6/10 (p/p) permet l'obtention d'un polyuréthanne solide de type PRC 1700. Les plaques sont réalisées selon deux coulées successives, la première avec polyols + DAP-2,8 tétraméthyl en fine couche ; la seconde sans DAP est coulée à froid après polymérisation complète de la première.

Une plaque de polyuréthanne contenant de la N,N,N',N'-Tétraméthyl-10H-phénothiazine-2,8-diamine (non covalente) ou de la 2-[(8-Dimethylamino-10H-phenothiazin-2-yl)-(2-hydroxy-ethyl)-amino]-ethanol (liaison covalente), réduit en 1 heure sous lumière blanche, plus de 1 log du titre infectieux d' *Escherichia coli* CIP 54 127. Le témoin à l'obscurité ne présente aucune activité dans les mêmes conditions.

## Revendications

1. Composé 2,8-diaminophénothiazine de formule (I) suivante : 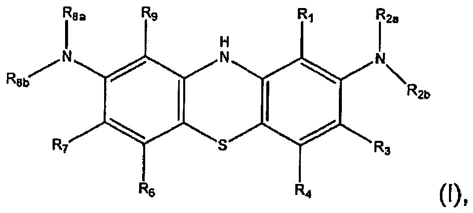 dans laquelle:
(i) les groupes R₁, R₃, R₄, R₆, R₇ et R₉ représentent chacun, indépendamment l'un de l'autre un groupe choisi parmi:
- un atome d'hydrogène,
- un halogène,
- un groupe alkyle ayant de 1 à 12 atomes de carbone non substitué,
- un groupe alkyle ayant de 1 à 12 atomes carbone substitué par un groupe phényle, ledit groupe phényle étant :
- soit non substitué,
- soit substitué par un ou plusieurs groupes choisis parmi un halogène, un hydroxy, un alkyle ayant de 1 à 12 atomes de carbone, et un alcoxy ayant de 1 à 12 atomes de carbone,
(ii) les groupes R₂ₐ, R_{2b}, R₈ₐ et R_{8b} représentent chacun, indépendamment l'un de l'autre un groupe choisi parmi :
- un atome d'hydrogène,
- un groupe alkyle, un groupe hydroxyalkyle ou un groupe alcoxy alkyle ayant de 1 à 12 atomes de carbone non substitué,
- un groupe alkyle ou un groupe alcoxyalkyle ayant de 1 à 12 atomes de carbone substitué par un groupe phényle, ledit groupe phényle étant :
- soit non substitué,
- soit substitué par un groupe choisi parmi
- un halogène,
- un hydroxy,
- un alkyle ayant de 1 à 12 atomes de carbone,
- un alcoxy ayant de 1 à 12 atomes de carbone,
- un alcényle ayant de 2 à 12 atomes de carbone, non substitué ou substitué par un groupe choisi parmi un halogène, un hydroxy, un alkyle ayant de 1 à 12 atomes de carbone, un alcoxy ayant de 1 à 12 atomes de carbone,
- un alcynyle ayant de 2 à 12 atomes de carbone, non substitué ou substitué par un groupe choisi parmi un halogène, un hydroxy, un alkyle ayant de 1 à 12 atomes de carbone, un alcoxy ayant de 1 à 12 atomes de carbone,
- un cycloalkyle ayant de 4 à 12 atomes de carbone non substitué ou substitué par un groupe choisi parmi un halogène, un hydroxy, un alkyle ayant de 1 à 12 atomes de carbone, un alcoxy ayant de 1 à 12 atomes de carbone,
- un cycloalcényle ayant de 4 à 12 atomes de carbone non substitué ou substitué par un groupe choisi parmi un halogène, un hydroxy, un alkyle ayant de 1 à 12 atomes de carbone, un alcoxy ayant de 1 à 12 atomes de carbone,
(iii) ou bien les groupes -NR₂ₐR_{2b} ou -NR₈ₐR_{8b} représentent, indépendamment l'un de l'autre, un hétérocycle ayant de 4 à 12 atomes de carbone, saturé ou insaturé, ledit hétérocycle pouvant comprendre un ou plusieurs hétéroatomes additionnels choisis parmi l'azote, l'oxygène et le soufre, ledit hétérocycle étant non substitué ou étant substitué par un groupe choisi parmi un halogène, un hydroxy et un alkyle ayant de 1 à 12 atomes de carbone,
ainsi que tout isomère optique, stéréoisomère, diastéréoisomère ou énantiomère d'un composé de formule (I),
ainsi que les sels, les hydrates et les solvates d'un composé de formule (I),
à l'exception des composés de formule (I) pour lesquels chacun des groupes choisis parmi R₁, R₂ₐ, R_{2b}, R₃, R₄, R₅, R₇, R₈ₐ, R_{8b} et R₉ représente un atome d'hydrogène.

2. Composé de formule (I) selon la revendication 1, **caractérisé en ce que** les groupes R₂ₐ et R_{2b} représentent chacun un atome d'hydrogène.

3. Composé de formule (I) selon l'une des revendications 1 et 2, **caractérisé en ce que** les groupes R₈ₐ et R_{8b} représentent chacun un atome d'hydrogène.

4. Composé de formule (I) selon la revendication 1, **caractérisé en ce qu'**au moins un et au plus quatre des groupes choisis parmi R₂ₐ, R_{2b}, R₈ₐ et R_{8b} représente un groupe méthyle.

5. Composé de formule (I) selon la revendication 1, **caractérisé en ce qu'**il est choisi parmi les composés suivants :
N,N,N',N'-Tétraméthyl-10H-phénothiazine-2,8-diamine
N,N-Diméthyl-[8-(4-méthylpipérazin-1-yl)-10H-phénothiazin-2-yl]amine
N, N-Diméthyl-(8-morpholin-4-yl-10H-phénothiazin-2-yl)amine
N,N-Diéthyl-N',N'-diméthyl-10H-phénothiazine-2,8-diamine
N,N-Diéthyl-[8-(4-méthyl-pipérazin-1-yl)-10H-phénothiazin-2-yl]amine
N, N-Diéthyl-(8-morpholin-4-yl-10H-phénothiazin-2-yl)amine
N,N-Diméthyl-N',N'-dipropyl-10H-phénothiazine-2,8-diamine
[8-(4-Méthyl-pipérazin-1-yl)-10H-phénothiazin-2-yl]-dipropylamine
(8-Morpholin-4-yl-10H-phénothiazin-2-yl)-dipropylamine
N,N-Dibutyl-N',N'-diméthyl-10H-phénothiazine-2,8-diamine
N,N-Dibutyl-[8-(4-méthylpipérazin-1-yl)-10H-phénothiazin-2-yl]amine
N,N-Dibutyl-(8-morpholin-4-yl-10H-phénothiazin-2-yl)amine
N,N-Diméthyl-N'-propyl-10H-phénothiazine-2,8-diamine
3,N,N,N',N'-Pentaméthyl-10H-phénothiazine-2,8-diamine
2-[N-(8-Diméthylamino-10H-phénothiazin-2-yl)-N-(2-hydroxyéthyl)amino]éthanol

6. Procédé pour la préparation d'un composé 2,8-diaminophénothiazine de formule (I) selon la revendication 1,
ledit procédé comprenant une étape (b) au cours de laquelle le composé diphénylamine de formule (II) suivant : 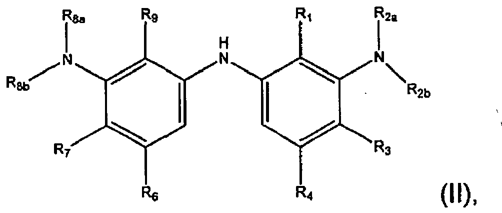 dans lequel les groupes R₁, R₂ₐ, R_{2b}, R₃, R₄, R₅, R₇, R₈ₐ, R_{8b} et R₉ ont ia même signification que pour le composé de formule (I),
est soumis à une étape de chauffage choisie parmi :
(b1) une étape de chauffage à une température allant de 60°C à la température d'ébullition du mélange réactionnel en présence d'iode et de soufre dans un solvant inerte pendant une durée allant de 1 heure à 1 nuit ; et
(b2) une étape de chauffage par micro-ondes pendant une durée allant de 10 secondes à six fois dix minutes ;
(b2-1) soit d'un mélange du composé de formule (II) avec de l'iode et du soufre, sans solvant ou en présence d'un solvant inerte ;
(b2-2) soit d'un mélange du composé de formule (II) avec de l'iode et du soufre, le composé de formule (II), l'iode et le soufre étant adsorbés sur un support.
afin d'obtenir le composé de formule (I).

7. Procédé selon la revendication 6, **caractérisé en ce qu'**il comprend une étape préalable (a) d'obtention d'un composé de formule (II) dans laquelle on fait réagir un composé de formule (III) suivante : 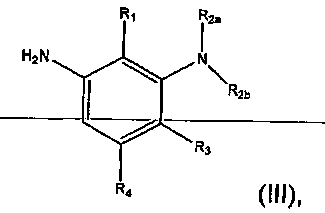 dans laquelle les groupes R₁, R₂ₐ, R_{2b}, R₃ et R₄ ont la même signification que dans la revendication 1,
avec un composé de formule (IV) suivante : 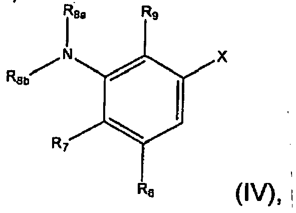 dans laquelle les groupes R₅, R₇,R₈ₐ, R_{8b} et R₉ ont la même signification que dans la revendication 1 et le groupe X représente un halogène ou un groupe sulfonate, en présence d'un catalyseur de palladium ou de nickel et d'un ligand de catalyseur et d'une base organique ou inorganique,
à une température allant de 80°C à 110°C et pendant une durée allant de 8 à 15 heures pour obtenir le composé de formule (II).

8. Procédé selon la revendication 7, **caractérisé en ce qu'**il comprend une étape préalable (a) d'obtention d'un composé de formule (II) dans laquelle on fait réagir un composé de formule (III) suivante : 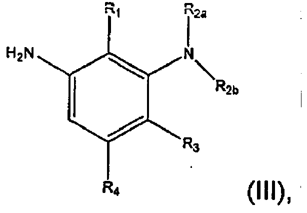 dans laquelle les groupes R₁, R₂ₐ, R_{2b}, R₃ et R₄ ont la même signification que dans la revendication1,
avec un composé de formule (IV) suivante : 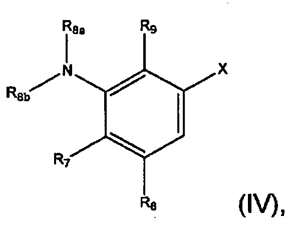 dans laquelle les groupes R₅, R₇,R₈ₐ, R_{8b} et R₉ ont la même signification que dans la revendication 1 et le groupe X représente un halogène ou un groupe sulfonate,
en présence d'un sel ou oxyde de cuivre et d'une base organique à une température allant de 40°C à la température d'ébullition du mélange réactionnel et pendant une durée allant de 2 heures à une nuit. pour obtenir le composé de formule (II).

9. Procédé selon l'une des revendications 6 à 8, dans lequel on obtient un composé de formule (I) dans laquelle au moins un des groupes choisis parmi R₁, R₂ₐ, R_{2b}, R₃, R₄, R₅, R₇,R₈ₐ, R_{8b} et R₉ ne représente pas un atome d'hydrogène est préparé à partir d'un composé de formule (I") dans laquelle chacun des groupes choisis parmi R₁, R₂ₐ, R_{2b}, R₃, R₄, R₅, R₇,R₈ₐ, R_{8b} et R₉ représente un atome d'hydrogène selon les étapes suivantes :
c) une étape réactionnelle choisie parmi une étape d'alkylation, une étape d'amination réductrice ou une étape d'acylation ; et
d) une étape de réduction.

10. Procédé selon l'une des revendications 6 à 8, dans lequel on obtient un composé de formule (I) dans laquelle au moins un des groupes choisis parmi R₁, R₂ₐ, R_{2b}, R₃, R₄, R₅, R₇,R₈ₐ, R_{8b} et R₉ ne représente pas un atome d'hydrogène est préparé à partir d'un composé de formule (I") dans laquelle chacun des groupes choisis parmi R₁, R₂ₐ, R_{2b}, R₃, R₄, R₅, R₇,R₈ₐ, R_{8b} et R₉ représente un atome d'hydrogène selon une étape choisie parmi :
- une réaction d'halogénation,
- une réaction de métallation dirigée,
- une réaction de Friedel, et
- une réaction de réarrangement.

11. Utilisation d'un composé de formule (I) selon l'une quelconque des revendications 1 à 5, en tant qu'agent biocide.

12. Utilisation selon la revendication 11, dans le domaine de la désinfection, l'épuration, la stérilisation ou la purification des fluides.

13. Utilisation selon la revendication 12, **caractérisée en ce que** les fluides sont choisis parmi les effluents industriels et les effluents domestiques.

14. Composition pour la désinfection, l'épuration, la stérilisation ou la purification des fluides comprenant un composé de formule (I) selon l'une des revendications 1 à 7.

15. Composition selon la revendication 14, se présentant sous la forme d'un support sur lequel, ou dans lequel, est immobilisé le composé de formule (I).

## Claims

1. A 2,8-diaminophenothiazine compound of following formula (I): 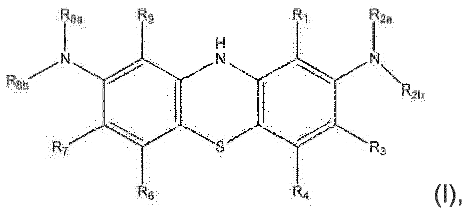 wherein:
(i) R₁, R₃, R₄, R₆, R₇ and R₉ groups each represent, independently from one another a group selected from:
- a hydrogen atom,
- a halogen,
- an alkyl group having from 1 to 12 unsubstituted carbon atoms,
- an alkyl group having from 1 to 12 carbon atoms substituted by a phenyl group, said phenyl group being:
- either unsubstituted,
- or substituted by one or more groups selected from a halogen, a hydroxy, an alkyl having from 1 to 12 carbon atoms, and an alkoxy having from 1 to 12 carbon atoms,
(ii) R₂ₐ, R_{2b}, R₈ₐ and R_{8b} groups each represent, independently from one another a group selected from:
- a hydrogen atom,
- an alkyl group, a hydroxyalkyl group or an alkoxyalkyl group having from 1 to 12 unsubstituted carbon atoms,
- an alkyl group or an alkoxyalkyl group having from 1 to 12 carbon atoms substituted by a phenyl group, said phenyl group being:
- either unsubstituted,
- or substituted by a group selected from
- a halogen,
- a hydroxy,
- an alkyl having from 1 to 12 carbon atoms,
- an alkoxy having from 1 to 12 carbon atoms,
- an alkenyl having from 2 to 12 carbon atoms, unsubstituted or substituted by a group selected from a halogen, a hydroxy, an alkyl having from 1 to 12 carbon atoms, an alkoxy having from 1 to 12 carbon atoms,
- an alkynyle having from 2 to 12 carbon atoms, unsubstituted or substituted by a group selected from a halogen, a hydroxy, an alkyl having from 1 to 12 carbon atoms, an alkoxy having from 1 to 12 carbon atoms,
- a cycloalkyl having from 4 to 12 carbon atoms, unsubstituted or substituted by a group selected from a halogen, a hydroxy, an alkyl having from 1 to 12 carbon atoms, an alkoxy having from 1 to 12 carbon atoms,
- a cycloalkenyl having from 4 to 12 carbon atoms, unsubstituted or substituted by a group selected from a halogen, a hydroxy, an alkyl having from 1 to 12 carbon atoms, an alkoxy having from 1 to 12 carbon atoms,
(iii) or -NR₂ₐR_{2b} or -NR₈ₐR_{8b} groups, independently from one another, represent a heterocycle having from 4 to 4 to 12 carbon atoms, either saturated or unsaturated, where said heterocycle may comprise one or more additional heteroatoms selected from nitrogen, oxygen and sulfur, said heterocycle being unsubstituted or being substituted by a group selected from a halogen, a hydroxy and an alkyl having from 1 to 12 carbon atoms,
as well as any optical isomer, stereoisomer, diastereoisomer or enantiomer of a compound of formula (I),
as well as salts, hydrates and solvates of a compound of formula (I),
except compounds of formula (I), wherein each of the groups selected from R₁, R₂ₐ, R_{2b}, R₃, R₄, R₅, R₇,R₈ₐ, R_{8b} and R₉ represents a hydrogen atom.

2. A compound of formula (I) according to claim 1, wherein R₂ₐ and R_{2b} groups each represent a hydrogen atom.

3. A compound of formula (I) according to claim 1 or 2, wherein R₈ₐ and R_{8b} groups each represent a hydrogen atom.

4. A compound of formula (I) according to claim 1, wherein at least one and at most four of the groups selected from R₂ₐ, R_{2b}, R₈ₐ and R_{8b} represent(s) a methyl group.

5. A compound of formula (I) according to claim 1, which is selected from following compounds:
N,N,N',N'-Tetramethyl-10H-phenothiazine-2,8-diamine
N,N-Dimethyl-[8-(4-methylpiperazin-1-yl)-10H-phenothiazin-2-yl]amine
N,N-Dimethyl-(8-morpholin-4-yl-10H-phenothiazin-2-yl)amine
N,N-Diethyl-N',N'-dimethyl-10H-phenothiazine-2,8-diamine
N,N-Diethyl-[8-(4-methyl-piperazin-1-yl)-10H-phenothiazin-2-yl]amine
N,N-Diethyl-(8-morpholin-4-yl-10H-phenothiazin-2-yl)amine
N,N-Dimethyl-N',N'-dipropyl-10H-phenothiazine-2,8-diamine
[8-(4-Methyl-piperazin-1-yl)-10H-phenothiazin-2-yl]-dipropylamine
(8-Morpholin-4-yl-10H-phenothiazin-2-yl)-dipropylamine
N,N-Dibutyl-N',N'-dimethyl-10H-phenothiazine-2,8-diamine
N,N-Dibutyl-[8-(4-methylpiperazin-1-yl)-10H-phenothiazin-2-yl]amine
N,N-Dibutyl-(8-morpholin-4-yl-10H-phenothiazin-2-yl)amine
N,N-Dimethyl-N'-propyl-10H-phenothiazine-2,8-diamine
3,N,N,N',N'-Pentamethyl-10H-phenothiazine-2,8-diamine
2-[N-(8-Dimethylamino-10H-phenothiazin-2-yl)-N-(2-hydroxyethyl)amino]ethanol

6. A method for preparing a 2,8-diaminophenothiazine compound of formula (I) according to claim 1, said method comprising a step (b) during which the diphenylamine compound of following formula (II): 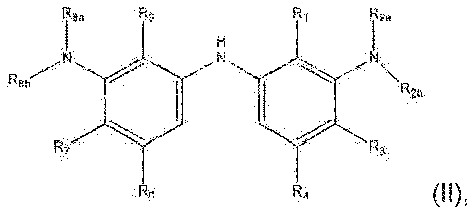 wherein R₁, R₂ₐ, R_{2b}, R₃, R₄, R₅, R₇,R₈ₐ, R_{8b} and R₉ groups have the same meaning as for compound of formula (I),
is submitted to a heating step selected from:
(b1) a heating step at a temperature ranging from 60°C to the boiling temperature of the reaction mixture in the presence of iodine and sulfur in an inert solvent for a time period ranging from 1 hour to a night; and
(b2) a microwave heating step for a time period ranging from 10 seconds to six times ten minutes;
(b2-1) of a combination of the compound of formula (II) with iodine and sulfur, with no solvent or in the presence of an inert solvent;
(b2-2) or of a combination of the compound of formula (II) with iodine and sulfur, the compound of formula (II), iodine and sulfur being adsorbed onto a support. so as to obtain the compound of formula (I).

7. A method according to claim 6, which comprises a previous step (a) for obtaining a compound of formula (II) wherein a compound of following formula (III): 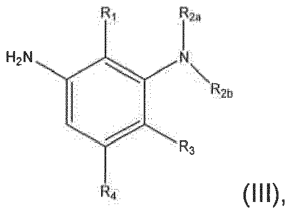 wherein R₁, R₂ₐ, R_{2b}, R₃ and R₄ groups have the same meaning as in claim 1, is reacted with a compound of following formula (IV): 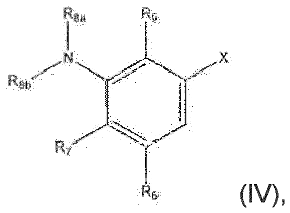 wherein R₅, R₇,R₈ₐ, R_{8b} and R₉ groups have the same meaning as in claim 1 and X represents a halogen or a sulfonate group,
in the presence of a palladium or a nickel catalyst and a catalyst ligand, together with an organic or inorganic base,
at a temperature ranging from 80°C to 110°C and for a time period ranging from 8 to 15 hours for obtaining the compound of formula (II).

8. A method according to claim 7, which comprises a previous step (a) for obtaining a compound of formula (II) wherein a compound of following formula (III): 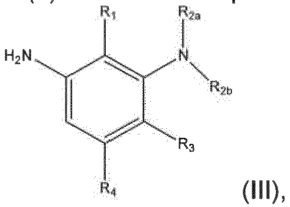 wherein R₁, R₂ₐ, R_{2b}, R₃ and R₄ groups have the same meaning as in claim 1, is reacted with a compound of following formula (IV): 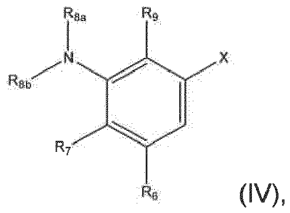 wherein R₅, R₇,R₈ₐ, R_{8b} and R₉ groups have the same meaning as in claim 1 and X represents a halogen or a sulfonate group,
in the presence of a salt or copper oxide and an organic base at a temperature ranging from 40°C to the boiling temperature of the reaction mixture and for a time period ranging from 2 hours to a night.
for obtaining the compound of formula (II).

9. A method according to anyone of claims 6 to 8, wherein a compound of formula (I) is obtained, wherein at least one of the groups selected from R₁, R₂ₐ, R_{2b}, R₃, R₄, R₅, R₇,R₈ₐ, R_{8b} and R₉ is not a hydrogen atom and is prepared from a compound of formula (I") wherein each of the groups selected from R₁, R₂ₐ, R_{2b}, R₃, R₄, R₅, R₇,R₈ₐ, R_{8b} and represents a hydrogen atom according the following steps:
c) a reaction step selected from an alkylation step, a reductive amination step or an acylation step; and
d) a reduction step.

10. A method according to anyone of claims 6 to 8, wherein a compound of formula (I) is obtained, wherein at least one of the groups selected from R₁, R₂ₐ, R_{2b}, R₃, R₄, R₅, R₇,R₈ₐ, R_{8b} and R₉ is not a hydrogen atom and is prepared from a compound of formula (I") wherein each of the groups selected from R₁, R₂ₐ, R_{2b}, R₃, R₄, R₅, R₇,R₈ₐ, R_{8b} and R₉ represents a hydrogen atom, according to a step selected from:
- a halogenation reaction,
- a directed metallation reaction,
- a Friedel reaction, and
- a rearrangement reaction.

11. Use of a compound of formula (I) according to anyone of claims 1 to 5, as a biocide agent.

12. Use according to claim 11, in the fluid disinfection, sterilization or purification field.

13. Use according to claim 12, wherein said fluids are selected from industrial effluents and domestic effluents.

14. A composition for disinfecting, sterilizing or purifying fluids comprising a compound of formula (I) according to anyone of claims 1 to 7.

15. A composition according to claim 14, in the form of a support onto which or in which the compound of formula (I) has been immobilized.

## Patentansprüche

1. 2,8-Diaminophenothiazinverbindung folgender Formel (I): 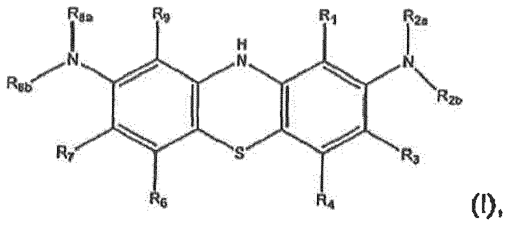 worin:
(i) die Gruppen R₁, R₃, R₄, R₆, R₇ und R₉ jeweils unabhängig voneinander eine Gruppe darstellen, die ausgewählt ist aus:
- einem Wasserstoffatom,
- ein Halogen,
- einer unsubstituierten Alkylgruppe mit 1 bis 12 Kohlenstoffatomen,
- einer Alkylgruppe mit 1 bis 12 Kohlenstoffatomen, die durch eine Phenylgruppe substituiert ist, wobei die Phenylgruppe:
- entweder unsubstituiert ist
- oder durch eine oder mehrere Gruppen, ausgewählt aus einem Halogen, einem Hydroxy, einem Alkyl mit 1 bis 12 Kohlenstoffatomen und einem Alkoxy mit 1 bis 12 Kohlenstoffatomen, substituiert ist,
(ii) die Gruppen R₂ₐ, R_{2b}, R₈ₐ und R_{8b} jeweils unabhängig voneinander eine Gruppe darstellen, die ausgewählt ist aus:
- einem Wasserstoffatom,
- ein Alkylgruppe, ein Hydroxyalkylgruppe oder einer Alkoxyalkylgruppe mit 1 bis 12 Kohlenstoffatomen, die unsubstituiert ist,
- einer Alkylgruppe oder ein Alkoxyalkylgruppe mit 1 bis 12 Kohlenstoffatomen, die durch eine Phenylgruppe substituiert ist, wobei die Phenylgruppe:
- entweder unsubstituiert ist
- oder durch eine Gruppe substituiert ist, die ausgewählt ist aus
- einem Halogen,
- einem Hydroxy,
- einem Alkyl mit 1 bis 12 Kohlenstoffatomen,
- eine Alkoxy mit 1 bis 12 Kohlenstoffatomen,
- einem Alkenyl mit 2 bis 12 Kohlenstoffatomen, das unsubstituiert ist oder substituiert ist durch eine Gruppe, ausgewählt aus eine Halogen, einem Hydroxy, einem Alkyl mit 1 bis 12 Kohlenstoffatomen, einem Alkoxy mit 1 bis 12 Kohlenstoffatomen,
- einem Alkynyl mit 2 bis 12 Kohlenstoffatomen, das unsubstituiert ist oder substituiert ist durch eine Gruppe, ausgewählt aus einem Halogen, einem Hydroxy, einem Alkyl mit 1 bis 12 Kohlenstoffatomen, einem Alkoxy mit 1 bis 12 Kohlenstoffatomen,
- eine Cycloalkyl mit 4 bis 12 Kohlenstoffatomen, das unsubstituiert ist oder substituiert ist durch eine Gruppe, ausgewählt aus einem Halogen, einem Hydroxy, einem Alkyl mit 1 bis 12 Kohlenstoffatomen, einem Alkoxy mit 1 bis 12 Kohlenstoffatomen,
- einem Cycloalkenyl mit 4 bis 12 Kohlenstoffatomen, das unsubstituiert ist oder substituiert ist durch eine Gruppe, ausgewählt aus einem Halogen, einem Hydroxy, einem Alkyl mit 1 bis 12 Kohlenstoffatomen, einem Alkoxy mit 1 bis 12 Kohlenstoffatomen,
(iii) oder aber die Gruppen -NR₂ₐ R_{2b} oder -NR₈ₐR_{8b} jeweils unabhängig voneinander einen Heterocyclus mit 4 bis 12 Kohlenstoffatomen, gesättigt oder ungesättigt, darstellen, wobei der Heterocyclus ein oder mehrere zusätzliche Heteroatome, ausgewählt aus Stickstoff, Sauerstoff und Schwefel umfassen kann, wobei der Heterocyclus unsubstituiert ist oder durch eine Gruppe, ausgewählt aus einem Halogen, einem Hydroxy und einem Alkyl mit 1 bis 12 Kohlenstofiatomen substituiert ist,
sowie jedwedes optische Isomer, Stereoisomer, Diastereoisomer oder Enantiomer einer Verbindung der Formel (I),
sowie die Salze, die Hydrate und die Solvate einer Verbindung der Formel (I),
mit Ausnahme der Verbindungen der Formel (I), bei denen eine jede der Gruppen, ausgewählt aus R₁, R₂ₐ, R_{2b}, R₃, R₄, R₅, R₇, R₈ₐ, R_{8b} und R₉, ein Wasserstoffatom darstellt.

2. Verbindung der Formel (I) nach Anspruch 1, **dadurch gekennzeichnet, dass** die Gruppen R₂ₐ und R_{2b} jeweils ein Wasserstoffatom darstellen.

3. Verbindung der Formel (I) nach einem der Ansprüche 1 und 2, **dadurch gekennzeichnet, dass** die Gruppen R₈ₐ und R_{8b} jeweils ein Wasserstoffatom darstellen.

4. Verbindung der Formel (I) nach Anspruch 1, **dadurch gekennzeichnet, dass** wenigstens eine und höchstens vier der Gruppen, ausgewählt aus R₂ₐ, R_{2b}, R₈ₐ und R_{8b}, eine Methylgruppe darstellt.

5. Verbindung der Formel (I) nach Anspruch 1, **dadurch gekennzeichnet, dass** sie aus den folgenden Verbindungen ausgewählt ist:
N,N,N',N'-Tetramethyl-10H-phenothiazin-2,8-diamin
N,N-Dimethyl-[8-(4-methylpiperazin-1-yl)-10H-phenothiazin-2-yl]amin
N,N-Dimethyl-(8-morpholin-4-yl-10H-phenothiazin-2-yl)amin
N,N-Diethyl-N',N'-dimethyl-10H-phenothiazin-2,8-diamin
N,N-Diethyl-[8-(4-methyl-piperazin-1-yl)-10H-phenothiazin-2-yl]amin
N,N-Diethyl-(8-morpholin-4-yl-10H-phenothiazin-2-yl)amin
N,N-Dimethyl-N',N'-dipropyl-10H-phenothiazin-2,8-diamin
[8-(4-Methyl-piperazin-1 -yl)-10H-phenothiazin-2-yl]-dipropylamin
(8-Morpholin-4-yl-10H-phenothiazin-2-yl)-dipropylamin
N,N-Dibutyl-N',N'-dimethyl-10H-phenothiazin-2,8-diamin
N,N-Dibutyl-[8-(4-methylpiperazin-1-yl)-10H-phenothiazin-2-yl]amin
N,N-Dibutyl-(8-morpholin-4-yl-10H-phenothiazin-2-yl)amin
N,N-Dimethyl-N'-propyl-10H-phenothiazin-2,8-diamin
3,N,N,N',N'-Pentamethyl-10H-phenothiazin-2,8-diamin
2-[N-(8-Dimethylamino-10H-phenothiazin-2-yl)-N-(2-hydroxyethyl)amino]ethanol.

6. Verfahren zur Herstellung einer 2,8-Diaminophenothiazinverbindung der Formel (I) nach Anspruch 1, wobei das Verfahren einen Schritt (b) umfasst, im Laufe dessen die Diphenylaminverbindung folgender Formel (II): 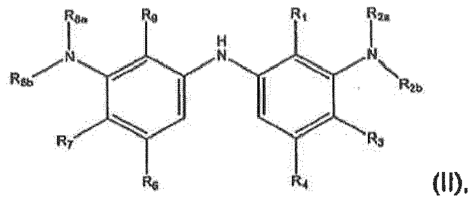 worin die Gruppen R₁, R₂ₐ, R_{2b}, R₃, R₄, R₅, R₇, R₈ₐ, R_{8b} und R₉ die gleiche Bedeutung wie bei der Verbindung der Formel (I) haben,
einem Erhitzungsschritt unterzogen wird, der ausgewählt ist aus:
(b1) einem Schritt zum Erhitzen auf eine Temperatur im Bereich von 60 °C bis zur Siedetemperatur des Reaktionsgemischs, in Anwesenheit von Jod und Schwefel, in einem inerten Lösungsmittel, über eine Dauer von 1 Stunde bis zu 1 Nacht, und
(b2) einem Schritt zum Erhitzen mittels Mikrowellen, über eine Dauer von 10 Sekunden bis zu sechs mal zehn Minuten;
(b2-1) entweder eines Gemischs der Verbindung der Formel (II) mit Jod und Schwefel, ohne Lösungsmittel oder in Anwesenheit eines inerten Lösungsmittels;
(b2-2) oder eines Gemischs der Verbindung der Formel (II) mit Jod und Schwefel, wobei die Verbindung der Formel (II), das Jod und der Schwefel an einem Träger adsorbiert werden, um die Verbindung der Formel (I) zu erhalten.

7. Verfahren nach Anspruch 6, **dadurch gekennzeichnet, dass** es einen vorherigen Schritt (a) für den Erhalt ein Verbindung der Formel (II) umfasst, bei dem man Verbindung folgender Formel (III): 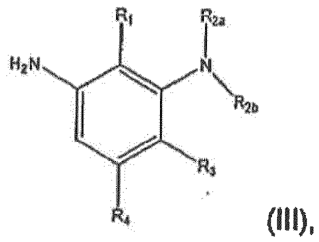 worin die Gruppen R₁, R₂ₐ, R_{2b}, R₃ und R₄ die gleiche Bedeutung wie in Anspruch 1 haben,
mit einer Verbindung folgender Formel (IV): 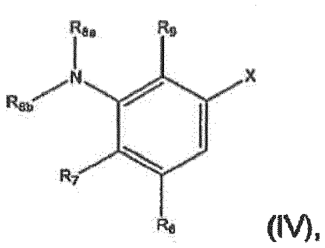 worin die Gruppen R₅, R₇, R₈ₐ, R_{8b} und R₉ die gleiche Bedeutung wie in Anspruch 1 haben und die Gruppe X ein Halogen oder eine Sulfonatgruppe darstellt,
in Anwesenheit eines Palladium- oder Nickel-Katalysators und eines Katalysatorliganden sowie einer organischen oder anorganischen Base,
bei einer Temperatur zwischen 80 °C und 110 °C und über eine Dauer von 8 bis 15 Stunden reagieren lässt, um die Verbindung der Formel (II) zu erhalten.

8. Verfahren nach Anspruch 7, **dadurch gekennzeichnet, dass** es einen vorherigen Schritt (a) für den Erhalt einer Verbindung der Formel (II) umfasst, bei dem man eine Verbindung folgender Formel (III): 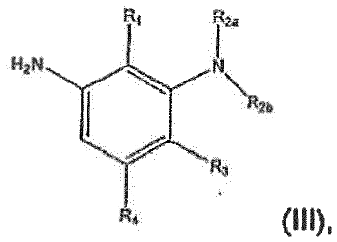 worin die Gruppen R₁, R₂ₐ, R_{2b}, R₃ und R₄ die gleiche wie in Anspruch 1 haben,
mit einer Verbindung folgender Formel (IV): 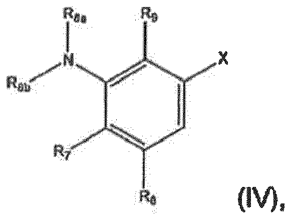 worin die Gruppen R₅, R₇, R₈ₐ, R_{8b} und R₉ die gleiche Bedeutung wie in Anspruch 1 haben und die Gruppe X ein Halogen oder eine Sulfonatgruppe darstellt,
in Anwesenheit eines Kupfersalzes oder -oxids sowie einer organischen Base,
bei einer Temperatur im Bereich von 40 °C bis zur Siedetemperatur des Reaktionsgemischs und über eine Dauer von 2 Stunden bis zu einer Nacht reagieren lässt, um die Verbindung der Formel (II) zu erhalten.

9. Verfahren nach einem der Ansprüche 6 bis 8, wobei eine Verbindung der Formel (I), worin wenigstens eine der Gruppen, ausgewählt aus R₁, R₂ₐ, R_{2b}, R₃, R₄, R₅, R₇, R₈ₐ, R_{8b} und R₉ kein Wasserstoffatom darstellt, aus einer Verbindung der Formel (I"), worin eine jede der Gruppen, ausgewählt aus R₁, R₂ₐ, _{2b}, R₃, R₄, R₅, R₇, R₈ₐ, R_{8b} und R₉ ein Wasserstoffatom darstellt, nach den folgenden Schritten hergestellt wird:
c) einem Reaktionsschritt, der aus einem Alkylierungsschritt, einem Schritt reduktiver Aminierung oder einem Acylierungsschritt ausgewählt ist, und
d) einem Reduktionsschritt.

10. Verfahren nach einem der Ansprüche 6 bis 8, wobei eine Verbindung der Formel (I), worin wenigstens eine der Gruppen, ausgewählt aus R₁, R₂ₐ, R_{2b}, R₃, R₄, R₅, R₇, R₈ₐ, R_{8b} und R₉ kein Wasserstoffatom darstellt, aus einer Verbindung der Formel (I"), worin eine jede der Gruppen, ausgewählt aus R₁, R₂ₐ, R_{2b}, R₃, R₄, R₅, R₇, R₈ₐ, R_{8b} und R₉ ein Wasserstoffatom darstellt, nach einem Schritt hergestellt wird, der ausgewählt ist aus:
- einer Halogenierungsreaktion,
- einer gerichteten Metallierungsreaktion,
- einer Friedel-Reaktion und
- einer Umlagerungsreaktion.

11. Verwendung einer Verbindung der Formel (I) nach einem der Ansprüche 1 bis 5, als biozides Mittel.

12. Verwendung nach Anspruch 11, auf dem Gebiet der Desinfektion, der Klärung, der Sterilisation oder der Reinigung von Fluiden.

13. Verwendung nach Anspruch 12, **dadurch gekennzeichnet, dass** die Fluide aus den Industrieabwässern und den Haushaltsabwässern ausgewählt sind.

14. Zusammensetzung für die Desinfektion, die Klärung, die Sterilisation oder die Reinigung von Fluiden, die eine Verbindung der Formel (I) nach einem der Ansprüche 1 bis 7 umfasst.

15. Zusammensetzung nach Anspruch 14, die in Form eines Trägers vorliegt, an dem, oder in dem, die Verbindung der Formel (I) festgelegt ist.
